# EUROPEAN PATENT APPLICATION

(11) **EP 2 649 995 A2**
(43) Date of publication of application: **16.10.2013**
(21) Application number: 13155998.1
(22) Date of filing: 27.02.2009
(51) Int. Cl.: A61K 31/395, A61K 31/47, A61K 31/731, A61K 31/663, A61K 39/395, A61P 35/00, A61K 41/00, C07K 16/28

(54) **Inhibition of post-radiation tumor growth**

(30) Priority: 29.02.2008 US 67652 P
(62) Divisional of application: 09716057.6
(71) Applicant: THE BOARD OF TRUSTEES OF THE LELAND STANFORD JUNIOR UNIVERSITY, Stanford, California 94305 (US)
(72) Inventor: Brown, John Martin, Redwood City, CA California 94061 (US); Ahn, G-one, Daly City, CA California 94015 (US); Kioi, Mitomu, Mountain View, CA California 94040 (US)
(74) Representative: Vossius & Partner

(57) **Abstract**

Compositions and methods for inhibiting the growth of solid tumors following radiation treatment are described. The compositions and methods target matrix metalloproteinases and bone marrow-derived cells expressing matrix metalloproteinases.

## Description

### CROSS REFERENCE TO RELATED APPLICATIONS

This application claims the priority of U.S. Provisional Patent Application No. 61/067,652 filed February 29,2008, incorporated herein by reference in its entirety.

### TECHNICAL FIELD

The present compositions and methods relate to inhibiting the growth of solid tumors following irradiation of tissue, which may be particularly relevant to the field of cancer treatment.

### BACKGROUND

### 1. Tumor growth

Tumor growth depends on the formation of new blood vessels through processes known as angiogenesis and vasculogenesis to supply oxygen and nutrients to neoplastic cells. Angiogenesis occurs primarily by endothelial migration and sprouting from preexisting blood vessels, while vasculogenesis involves the formation of blood vessels *in situ* by recruitment of precursor cells such as bone marrow (BM)-derived endothelial progenitor cells (EPCs) from the circulation. Although the contribution of EPCs to vasculogenesis has been demonstrated in several models including hind limb ischemia (Takahashi, T. *et al.* (1999) Nat. Med. *5*:434-38), vascular trauma (Gill, M. et al. (2001) Circ. Res. 88:167-74), and tumor growth (Lyden, D. et al. (2001). Nat. Med. 7:1194-201), the extent to which EPCs are incorporated into newly formed blood vessels in tumors varies significantly depending on the particular tumor model (De Palma, M. et al. (2003) Nat. Med. 9:789-95; Gothert, J.R. et al. (2004) Blood 104:1769-77; Lyden, D. et al. (2001). Nat. Med. 7:1194-201).

### 2. BM-derived myelomonocytic cells

BM-derived myelomonocytic cells expressing vascular endothelial growth factor receptor-1 (VEGFR-1) are also associated with tumor vasculature (De Palma, M. et al. (2003) Nat. Med. 9:789-95; Lyden, D. et al. (2001) Nat. Med. 7:1194-201). These cells are believed to be derived from precursor cell known as hemangioblasts (Rafii, S. et al. (2002) Nat. Rev. Cancer 2:826-35), which are myelomonocytic cells that share characteristics with EPCs, including the expression of platelet-endothelial cell adhesion molecule -1 (PECAM-1; CD31), Tie-2, endoglin, and integrate lectin and acetylated low density lipoprotein (Fujiyama, S. et al. (2003) Circ Res 93, 980-89; Rafii, S. et al. (2002) Nat. Rev. Cancer 2:826-35; Rohde, E. et al. (2006) Stem Cells 24:357-67). Functionally, BM-derived myelomonocytic cells have been shown to mimic EPCs in improving neovascularization in models of normal tissue injury (Capoccia, B.J. et al. (2006) Blood 108:2438-45; Fujiyama, S. et al. (2003) Circ Res 93, 980-89; Moldovan, N.I. et al. (2000) Circ. Res. 87:378-84). Myelomonocytic cells are often observed in the perivascular regions of the endothelium (De Palma, M. et al. (2003) Nat. Med. 9:789-95; Moldovan, N.I. et al. (2000) Circ. Res. 87:378-84) or co-localized with endothelial cells (Bailey, A.S., et al. (2006) Proc. Natl. Acad. Sci. U.S.A. 103:13156-61; Capoccia, B.J. et al. (2006) Blood 108:2438-45; Ruzinova, M.B. et al. (2003) Cancer Cell 4, 277-89), and have been shown to stabilize the tumor vasculature (Lyden, D. et al. (2001). Nat. Med. 7:1194-201). When depleted using the *Id1*^{+/-}*Id3*^{-/-} mouse model (where mobilization of VEGFR-1 and VEGFR-2 BM cells are genetically impaired (Lyden, D. et al. (2001) Nat. Med. 7:1194-201), or by using a suicide gene therapy approach (De Palma, M. et al. (2003) Nat. Med. 9:789-95), tumor growth is markedly inhibited, indicating that BM-derived myelomonocytic cells play an important role in tumor vasculogenesis.

BM-derived myelomonocytic cells that infiltrate tumors and differentiate into macrophages are commonly referred to as tumor-associated macrophages (TAMs) and clinical data indicate that the presence of large numbers of TAMs correlates with poor prognosis in cancers (Pollard, J.W. (2004) Nat. Rev. Cancer 4:71-78). TAMs are a polarized population of macrophages (Sica, A. et al. (2006) Eur. J. Cancer. 42:717-27) and release many angiogenic factors including vascular endothelial growth factor (VEGF), interleukin-8, tumor necrosis factor-α, and matrix metalloproteinase-9 (MMP-9) (Dirkx, A.E. et al. (2006) J. Leukoc. Biol. 80:1183-96; Lewis, C.E., and Pollard, J.W. (2006) Cancer Res. 66:605-12; Yang, L. et al. (2004) Cancer Cell 6:409-21).

### 3. Matrix metalloproteinase-9

MMP-9 is a member of a family of zinc containing endopeptidases involved in degradation of extracellular matrix (ECM) and in vascular remodeling (Heissig, B. et al. (2003) Curr. Opin. Hematol. 10:136-41). As with other members of the MMP family, MMP-9 is synthesized as an inactive zymogen (pro-MMP-9) that is activated by proteolysis or autolysis (Bergers, G. and Coussens, L.M. (2000) Curr. Opin. Genet. Dev. 10:120-27; Galis, Z.S. and Khatri, J.J. (2002) Circ. Res. 90:251-62). MMP-9 is involved in mobilizing EPCs and other progenitor cells of BM origin (Heissig, B. et al. (2002) Cell 109:625-37), liberating growth factors including VEGF (Bergers, G. et al. (2000) Nat. Cell. Biol. 2:737-44) and transforming growth factor-β (Yu, Q. and Stamenkovic, I. (2000) Genes Dev. 14:163-76) from the matrix-bound forms, and recruiting the BM-derived leukocytes to the tumor vasculature (Jodele, S. et al. (2005) Cancer Res. 65:3200-08). MMP-9 from BM-derived cells has been shown to initiate the angiogenic switch that leads to tumor growth and progression in K14-HPV16 epithelial squamous carcinoma in mice (Coussens, L.M. et al. (2000) Cell 103:481-90.; Giraudo, E. et al. (2004) J. Clin. Invest. 114:623-633).

MMP-9 is known to cleave fibrillar type-I collagen, which is the major constituent of the extracellular matrix to which endothelial cells are exposed in injured tissue (Seandel, M. et al. (2001) Blood 97:2323-32). Moreover MMP-9 provided by BM-derived macrophages has been shown to be essential in capillary branching in ischemia-induced revascularization of normal tissues (Johnson, C. et al. (2004) Circ. Res. 94:262-68). MMP-9 may also enhance local angiogenesis in a spatiotemporal manner due to its ability to release membrane-bound VEGF (Bergers, G. et al. (2000) Nat. Cell. Biol. 2:737-44), a growth factor critical for survival and growth of endothelial cells (Ferrara, N. et al. (2003) Nat. Med. 9:669-76).

### 4. Radiation therapy and the regrowth of tumors

Radiation therapy is one of the most important treatment modalities for cancer; however, many patients treated with radiation therapy relapse, as evidenced by regrowth of tumors at the irradiated site (Liang, B.C. et al. (1991) J. Neurosurg. 75:559-63). Regrowth of the tumor is puzzling because even if some tumor cells are not killed by irradiation, it seems unlikely that a sufficient number of endothelial cells survive to allow tumor regrowth (Itasaka, S. et al. (2007) Int. J. Rad. Oncol. Biol. Phys. 67:870-78; Tsai, J. et al. (2005) Cancer Biol. Ther. 4:1395-1400). One explanation for tumor regrowth is that a subset of BM-derived cells infiltrate an irradiated tumor and restore the vasculature by vasculogenesis. EPCs have recently been shown to rescue tumor growth following treatment by vascular disrupting agents (Shaked, Y. et al. (2006) Science 313:1785-87), although it remains to be determined whether similar events occur following irradiation of tumor sites. Solid tumors develop regions of hypoxia leading to an induction of the transcription factor HIF-1 (Bertout, J.A., et al. (2008). Nat. Rev. Cancer 8:967-75), which has been shown to be a major player in the recruitment of bone marrow derived cells (BMDC) to tumors including GBM (Du, R., et al. (2008) Cancer Cell 13:206-20) and to ischemic regions of normal tissues (Ceradini, D.J., et al. (2004) Nat. Med. 10:858-64). Importantly, tumors regrowing after irradiation or transplanted into an irradiated tissue are more hypoxic than control tumors (Sasai, K., and Brown, J.M. (1994) Int. J. Radiat. Oncol. Biol. Phys. 30:355-61; Zips, D., et al. (2001) Int. J. Radiat. Biol. 77:1185-93) and local tumor irradiation has been shown to induce HIF-1 (Moeller, B.J. et al. (2004) Cancer Cell 5:429-41)

The need exists for compositions and method that prevent tumor regrowth in irradiated tissues by, for example, inhibiting vasculogenesis.

### SUMMARY

The following aspects and embodiments thereof described and illustrated below are meant to be exemplary and illustrative, not limiting in scope.

In one aspect, a method for inhibiting post-irradiation tumor growth in a subject is provided, comprising
administering to the subject an amount of an inhibitor ofvasculogenesis selected from the group consisting of a matrix metalloproteinase (MMP) inhibitory compound, an agent that inactivates bone marrow (BM)-derived cells that express matrix metalloproteinase, and a HIF-1α inhibitor,
wherein said administering occurs before, during, or after a subject is exposed to radiation therapy, and wherein said amount is a therapeutically effective to prevent or inhibit regrowth of a tumor after the subject is exposed to radiation therapy.

In one aspect, the inhibitor of vasculogenesis is an MMP inhibitory compound.

In one aspect, a method for treating a solid tumor in a patient is provided, comprising:
exposing the solid tumor to radiation therapy; and
administering to the patient a matrix metalloproteinase (MMP) inhibitory compound.

In another aspect, the MMP inhibitor compound is selected from the group consisting of an antibody or fragment thereof and a small molecule.

In some embodiments, the MMP inhibitory compound is an antibody or fragment, thereof.

In some embodiments, the MMP inhibitory compound is a small molecule.

In some embodiments, the MMP inhibitory compound is selective for MMP-9.

In some embodiments, the solid tumor is a head or neck tumor. In some embodiments, the solid tumor is a tumor of the mouth. In some embodiments, the solid tumor is a glioblastoma.

In some embodiments, the MMP inhibitory compound is administered systemically. In some embodiments, the MMP inhibitory compound is administered locally tissue at the site of a tumor. In some embodiments, the MMP inhibitory compound is administered intratumorally.

In some embodiments, the MMP inhibitory compound is administered before, during, or after the step of exposing the tumor to radiation therapy.

In another aspect, the inhibitor of vasculogenesis is an agent that inactivates BM-derived cells that express matrix metalloproteinase.

In another aspect, a method for treating a solid tumor in a patient is provided, comprising:
exposing the solid tumor to radiation therapy; and
administering to the patient an agent that inactivates bone marrow (BM)-derived cells that express a matrix metalloproteinase (MMP).

In some embodiments, the BM-derived cells that express a matrix metalloproteinase in the tumors are CD11b-expressing myelomonocytic cells.

In some embodiments, the agent that inactivates BM-derived cells that express a matrix metalloproteinase is an antibody.

In some embodiments, the antibody is specific for CD11b. In some embodiments, the antibody is specific for CXCR4. In some embodiments the antibody is specific for Gr-1. In some embodiments, the antibody is specific for CD18.

In some embodiments, the agent that inactivates bone marrow (BM)-derived cells inhibits CXCR4. In some embodiments, the agent that inactivates BM-derived cells inhibits CXCR4 and SDF1 binding. In some embodiments, the agent is the bicyclam inhibitor AMD3100.

In some embodiments, the MMP is MMP-9.

In some embodiments, the solid tumor is a head or neck tumor. In some embodiments, the solid tumor is a tumor of the mouth. In some embodiments, the solid tumor is a glioblastoma.

In some embodiments, the agent that inactivates bone marrow (BM)-derived cells is administered systemically. In some embodiments, the agent that inactivates bone marrow (BM)-derived cells is surgically implanted underneath the skin. In some embodiments, the agent that inactivates bone marrow (BM)-derived cells is administered intratumorally.

In some embodiments, the agent that inactivates bone marrow (BM)-derived cells is administered before, during, or after the step of exposing the tumor to radiation therapy.

In another aspect, a pharmaceutical composition is provided, comprising an inhibitor of vasculogenesis selected from the group consisting of a matrix metalloproteinase (MMP) inhibitory compound, an agent that inactivates bone marrow (BM)-derived cells that express matrix metalloproteinase, and a HIF-1α inhibitor

In another aspect, a pharmaceutical composition is provided, comprising an agent that inactivates bone marrow (BM)-derived cells that express a matrix metalloproteinase (MMP).

In some embodiments, the agent is an antibody. In a particular embodiment, the antibody is specific for CD11b+ myelomonocytic cells. The MMP may be MMP-9.

In another aspect, a pharmaceutical composition is provided, comprising an inhibitor of a matrix metalloproteinase.

In some embodiments, the inhibitor is an antibody. In a particular embodiment, the antibody is specific for MMP-9.

In some embodiment, the agent is a HIF-1α inhibitor.

In one embodiment, the HIF-1α inhibitor is NSC134754.

These and other objects and features of the invention will be more fully appreciated when the following detailed description of the invention is read in conjunction with the accompanying drawings.

### BRIEF DESCRIPTION OF THE DRAWINGS

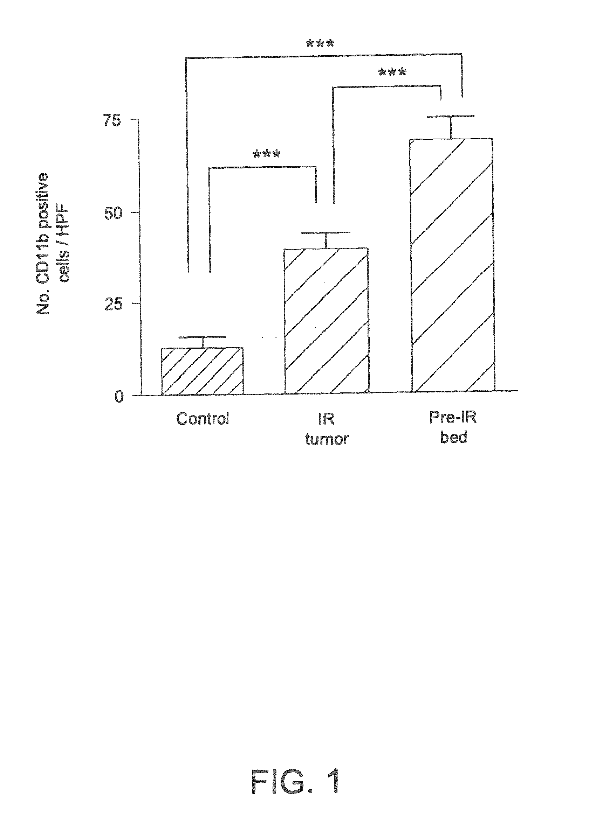

Figure 1 shows the results of immunostaining experiments in which tumor tissues were stained with various antibody-conjugates to determine the identity and localization of BM-derived CD11b+ myelomonocytic cells recruited to MT1A2 tumors grown on the irradiated tissues of FVB mice. Figure 1 is a graph showing the numbers of CD11b+ myelomonocytic cells in tumors grown on control animals, irradiated tumors (IR tumor), or tumors grown on irradiated tissues (pre-IR bed) for MT1A2. . Symbols and error bars represent the mean ± SEM for n ≥ 4 animals per group. *, **, and *** indicates *P* values of < 0.05, < 0.01, and < 0.001, respectively, determined by one-way ANOVA.

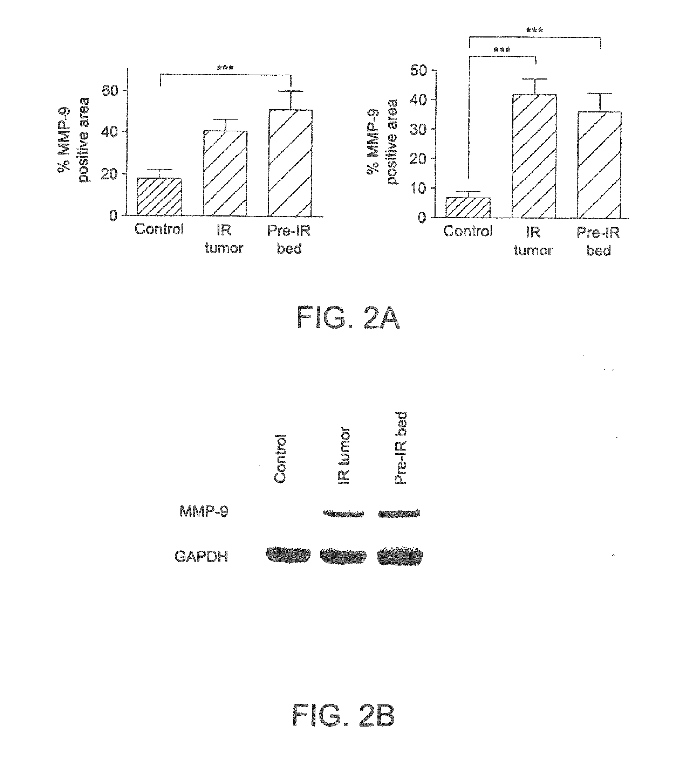

Figures 2A-2B show the results of experiments relating to the expression of MMP-9 in CD11b+ myelomonocytic cells present in tumors. Figure 2A is a graph showing the % MMP-9+ cells determined by the point-count method (left) and the number of CD11b and MMP-9 double positive cells in non-necrotic regions (right) of tumors. Symbols and error bars are mean ± SEM for n ≥ 4 animals per group. *, **, and *** denote for *P* values < 0.05, < 0.01, < 0.001, respectively, by one-way ANOVA. Figure 2B shows the results of an immunoblot for detecting MMP-9 in tumors. GAPDH was used as a loading control.

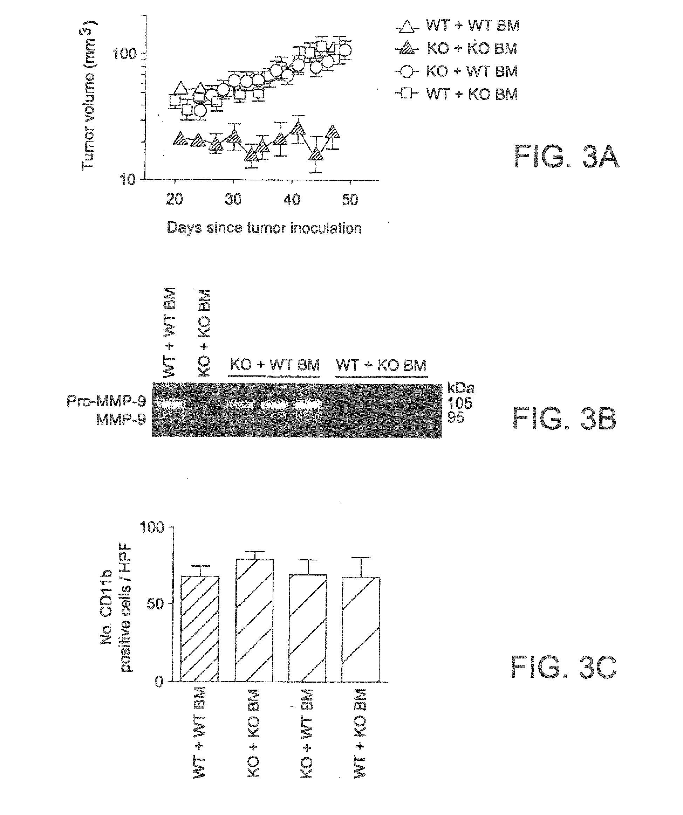

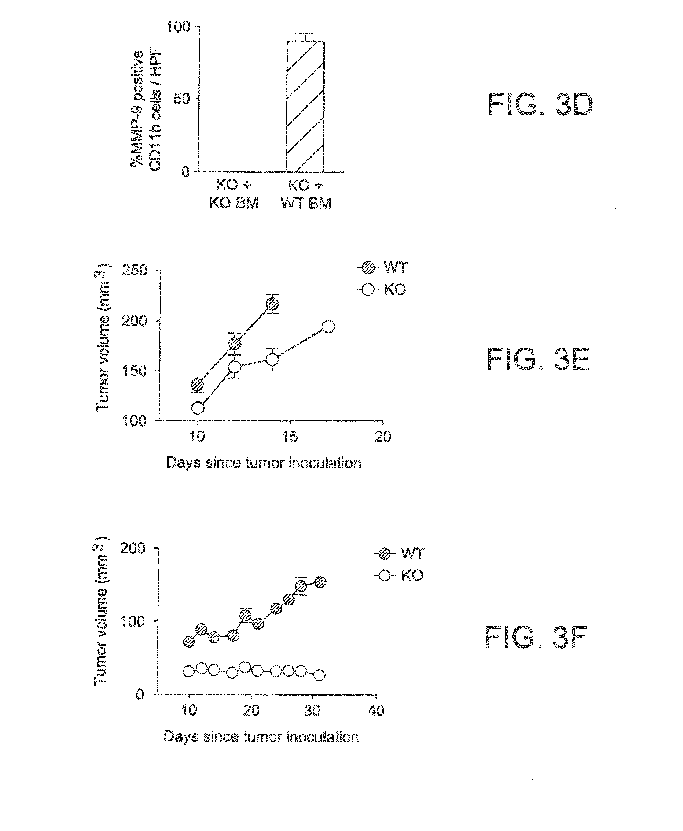

Figures 3A-3F show the results of experiment demonstrating that functional BM cells restore tumor growth in pre-irradiated tissues of MMP-9 KO mice. Figure 3A is a graph showing tumor growth rate in pre-irradiated tissues of (i) WT mice that had been irradiated and received transplanted BM cells from WT mice (WT + WT BM), *MMP-9* KO mice receiving BM cells from *MMP-9* KO mice (KO + KO BM), *MMP-9* KO mice receiving BM cells from WT mice (KO + WT BM), and WT mice receiving BM cells from *MMP-9* KO mice (WT + KO BM). Figure 3B shows the results of a zymography experiment in which BM cells of BM transplant recipients were isolated ≥ 4 weeks following BM transplantation and analyzed for MMP-9 activity. Each lane represents BM cells from one mouse. Figure 3C is a graph showing quantification of CD11b+ myelomonocytic cells from immunostaining for CD31 and α-SMA in tumors grown in wild-type or *MMP-9* KO mice. Figure 3D is a graph showing quantification of MMP-9 and CD11b double positive cells in tumors from KO + KO BM or KO+ WT BM. Symbols in A, C, and D are the mean ± SEM for n ≥ 5 mice per group. Figures 3E and 3F are graphs showing tumor growth rate on non-irradiated or irradiated tissues, respectively, of WT or KO mice.

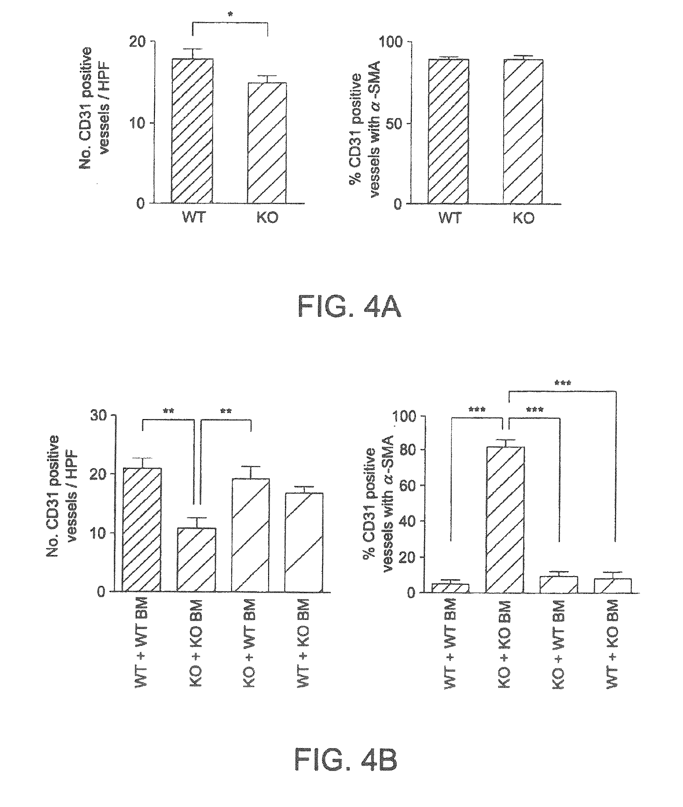

Figures 4A and 4B show the results of experiments demonstrating that immature blood vessels develop in tumors grown on pre-irradiated tissues of *MMP-9* KO + WT BM mice. Figure 4A is a graph showing the number of CD31+ vessels (left) and proportions of CD31+ vessels associated with α-SMA (right) in the tumors grown in wild-type (WT) or MMP-9 knock-out (KO) mice. Figure 4B includes a graph showing quantification of CD31 positive vessels (left panel) and the proportion of CD31+ vessels associated with α-SMA (right panel) in immunostained tumors grown in wild-type (WT) or MMP-9 knock-out (KO) mice. Symbols are the mean ± SEM for n ≥ 5 animals per group. *, **, and *** denote for P-values < 0.05, < 0.01, < 0.001, respectively, determined by one-way ANOVA.

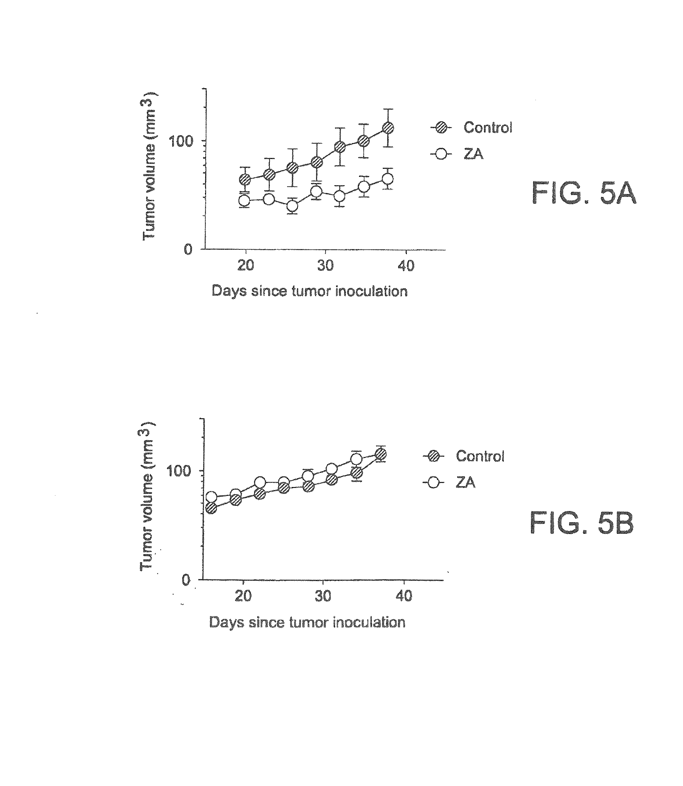

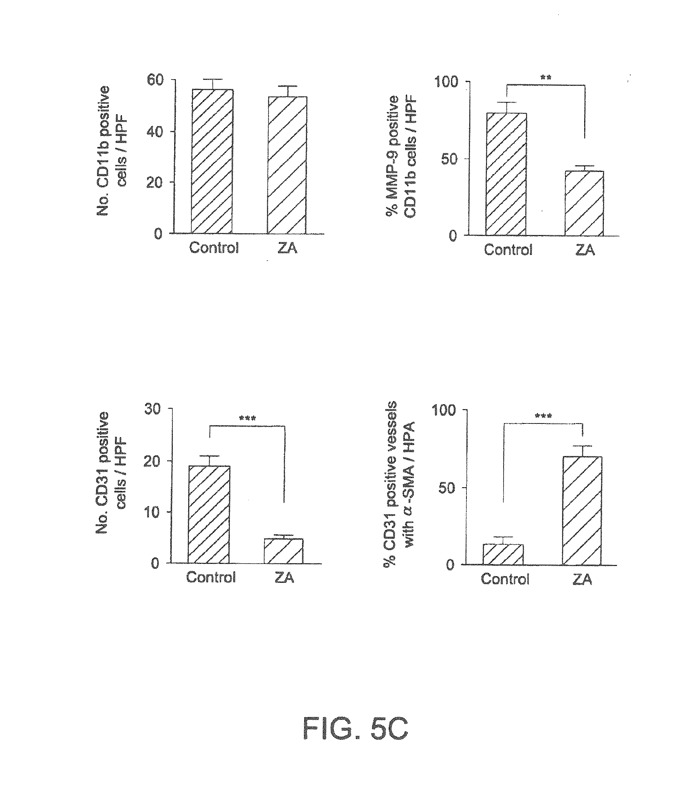

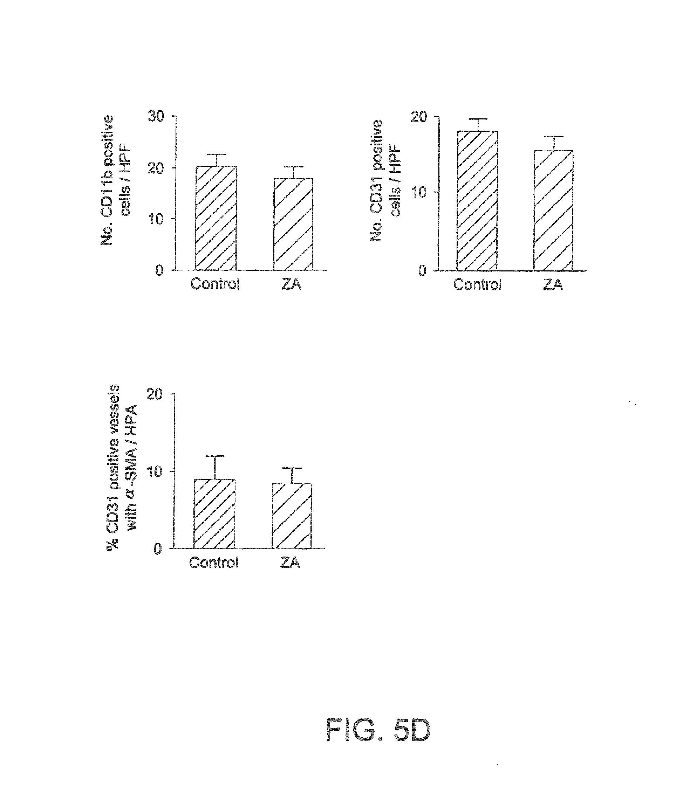

Figures 5A-5D show the results of experiment involving the pharmacological inhibition of MMP-9 in CD11b+ myelomonocytic cells by ZA. Figure 5A is a graph showing MT1A2 tumor growth in the presence (ZA, n = 5) or absence of ZA (control, n = 4) on pre-irradiated tissues of *MMP-9* KO mice that had received BM cells from WT mice. Figure 5B is a graph showing MT1A2 tumor growth on pre-irradiated tissues of WT mice receiving BM cells from *MMP-9* KO mice. n = 5 animals per group. Symbols and error bars in Figures 5A and 5B are the mean ± SEM.. Figure 5C includes a series of graphs showing quantification of the number of CD11b+ cells (upper left), the percentage of MMP-9 expressing CD11b+ cells (upper right), the number of CD31+ endothelial cells (lower left), and the number CD31 positive vessels that were associated with α-SMA (lower right) in the tumors from Figure 5A. Figure 5D includes a series of graphs showing quantification as in Figure 5C but from the tumors of Figure 5B. Error bars in Figures 5C and 5D are SEM. ** and *** denote for *P-*values < 0.01 and < 0.001, respectively, determined by two-tailed Student's *t*-test.

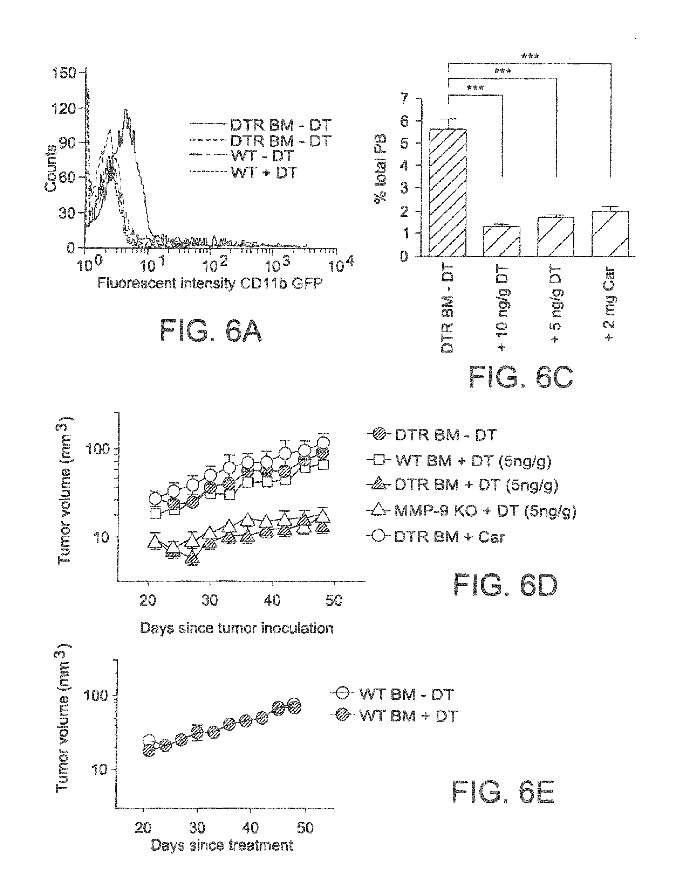

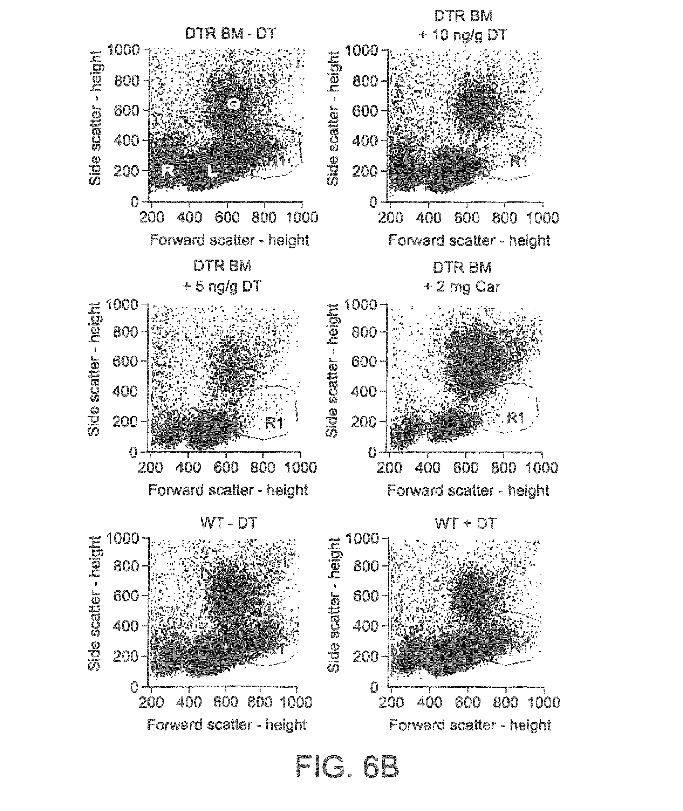

Figures 6A-6E show the results of experiments involving the depletion of CD11b+ cells expressing MMP-9 by diphtheria toxin (DT) in pre-irradiated tissues of *MMP-9* KO + DTR BM mice. Figure 6A is a graph showing the characteristics of peripheral blood from *MMP-9* KO mice transplanted with BM cells from transgenic mice expressing the diphtheria toxin receptor (DTR) and GFP under control of the CD11b promoter (DTR BM) or of WT mice without BM transplantation (WT). Figure 6B shows scatter graphs based on the signals produced by peripheral blood cells obtained from *MMP-9* KO + DTR BM (DTR BM) mice treated as indicated. Abbreviations: R, red blood cells; L, lymphocytes; G, granulocytes; M, monocytes. Figure 6C is graph showing quantification of red-gated monocytes from Figure 6B. Symbols and error bars indicate the mean ± SEM for n ≥ 5 mice per group. *** indicates *P* < 0.001 analyzed by one-way ANOVA. Figure 6D and 6E are graphs showing MT1A2 tumor growth on pre-irradiated tissues of *MMP-9* KO + DTR BM (DTR BM) mice (Figure 6D) and WT mice (Figure 6E) in the absence (-DT; n = 4) or presence (DT; 5 ng/g, n = 5) of DT. Tumors were also grown on pre-irradiated tissues of WT mice receiving BM cells from WT mice (WT BM, n = 5) or *MMP-9* KO mice (*MMP-9* KO, n = 5) that were treated with DT (5 ng/g). Error bars indicate the SEM for the number of animals indicated in the parentheses.

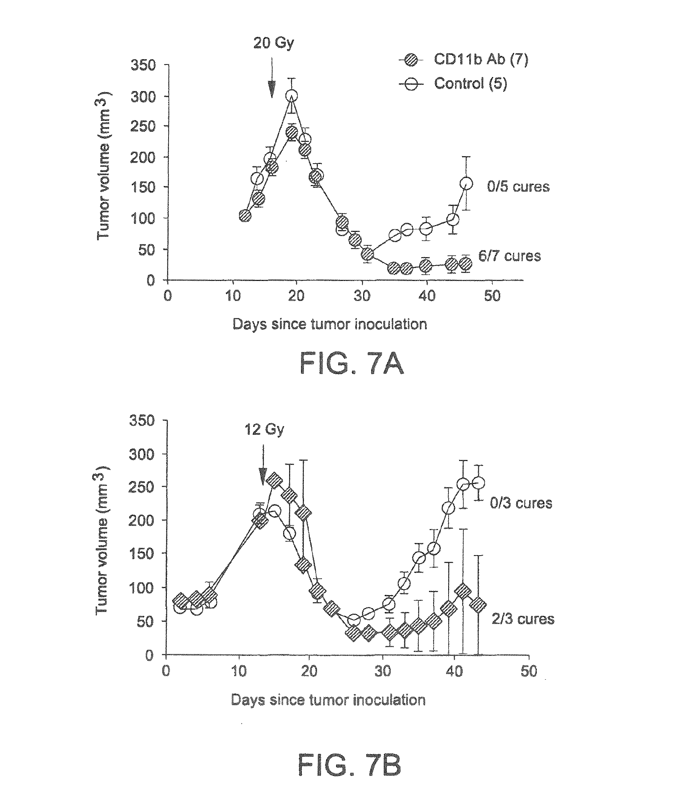

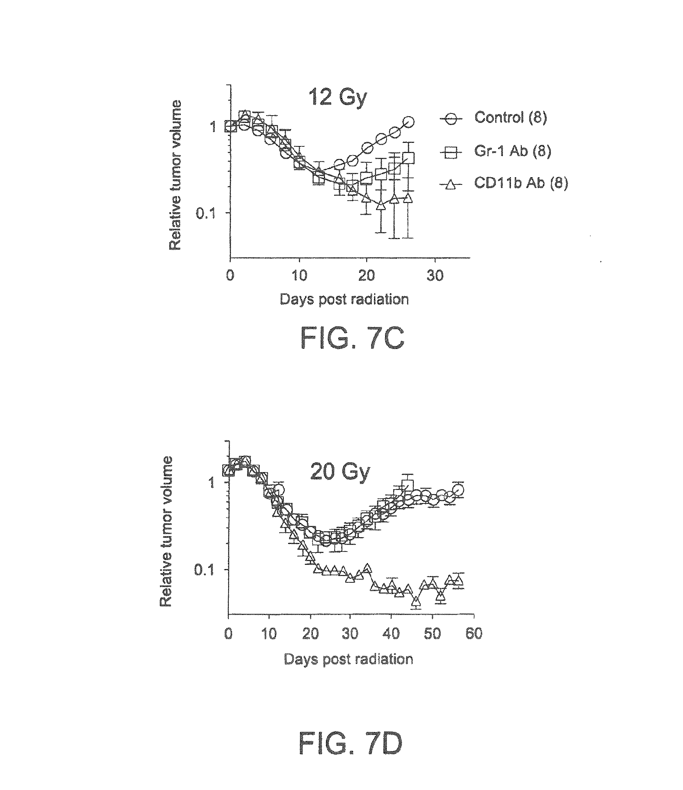

Figures 7A and 7B are graphs showing the results of experiments involving the inactivation of CD11b+ cells expressing MMP-9 using a neutralizing antibody specific for CD11b+. Figures 7C and 7D are graphs showing the results of experiments involving the inability of a neutralizing antibody specific for Gr-1 to inhibit CD11b+ mediated post-radiation tumor growth. FaDu (mouth cancer) cells were implanted on mice receiving the neutralizing CD11b+ antibody, or vehicle only (as a control), followed by irradiating the tumor site with 20 Gy (Figure 7A, 7C) or 12 Gy (Figure 7B, 7D), and allowing the tumors to regrow. Symbols and error bars indicate the mean ± SEM for the number of animals indicated in the parentheses.

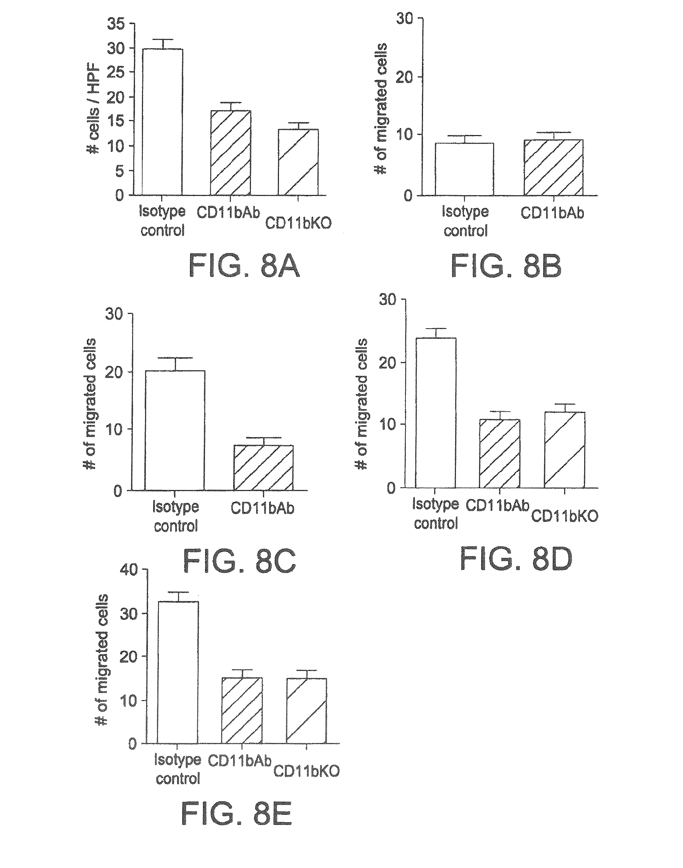

Figures 8A-8E are graphs showing the results of in vitro experiments involving inactivation of attachment to ECM by CD11+ cells expressing MMP-9 using a neutralizing antibody specific for CD11b+. Figure 8A is a graph showing inhibition of attachment by CD11+ cells by a neutralizing antibody specific for CD11+. Figures 8B-8E are graphs showing inhibition of chemotaxis by CD11+ cells by a neutralizing antibody specific for CD11+. In Figure 8B, no chemotaxis was induced. In Figures 8C, 8D, and 8E, chemotaxis was induced with 10% serum, VEGF, and M-CSF, respectively. Error bars indicate the SEM for four samples per group.

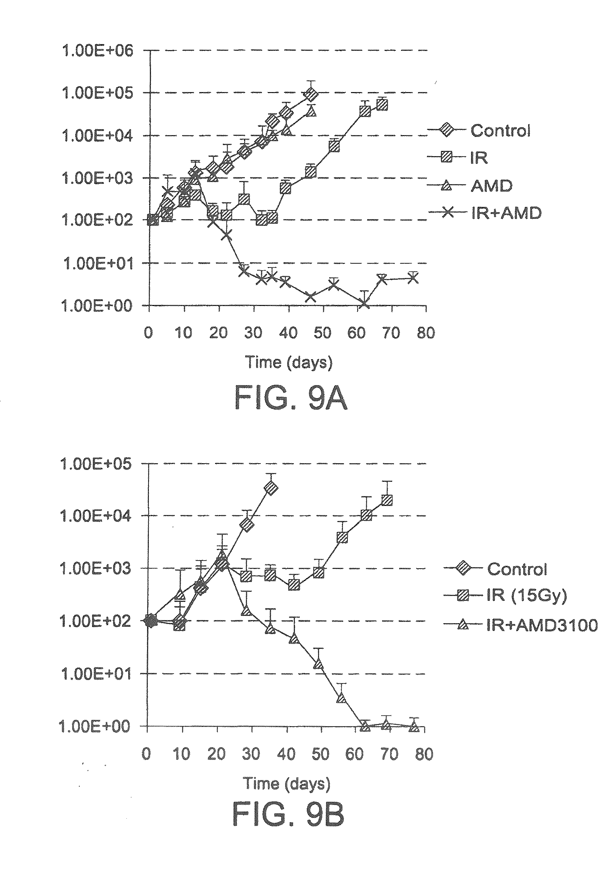

Figures 9A and 9B are graphs showing the results of experiments involving the inactivation of CD11b+ cells expressing MMP-9 using the CXCR4/SDF-1 bicyclam inhibitor AMD3100. Glioblastoma tumor cells were implanted on mice that were untreated (control) or that received either or both AMD3100, and irradiation under different conditions, after which time the tumors were then allowed to regrow. Figure 9A is a graph showing an early tumor model in which animals received 2Gy irradiation daily for five days and/or AMD3100 daily for three weeks starting 11 days after implantation. Figure 9B is a graph showing an advanced tumor model in which animals received 15Gy irradiation and/or AMD3100 daily for three weeks starting 21 days after implantation. Symbols and error bars indicate the mean ± SEM for five animals per group.

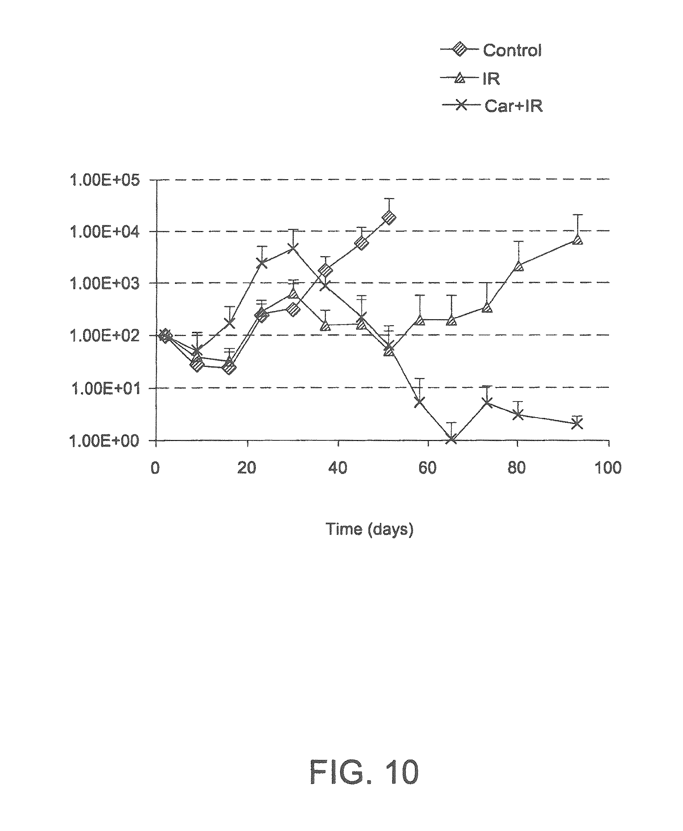

Figure 10 shows the growth of glioblastoma tumors that remained untreated (control) or that were either or both treated with 15 Gy irradiation (IR)on day 26 and depleted of CD11b+ myelomonocytic cells with carrageenan (Car). Symbols and error bars indicate the mean ± SEM for five animals per group.

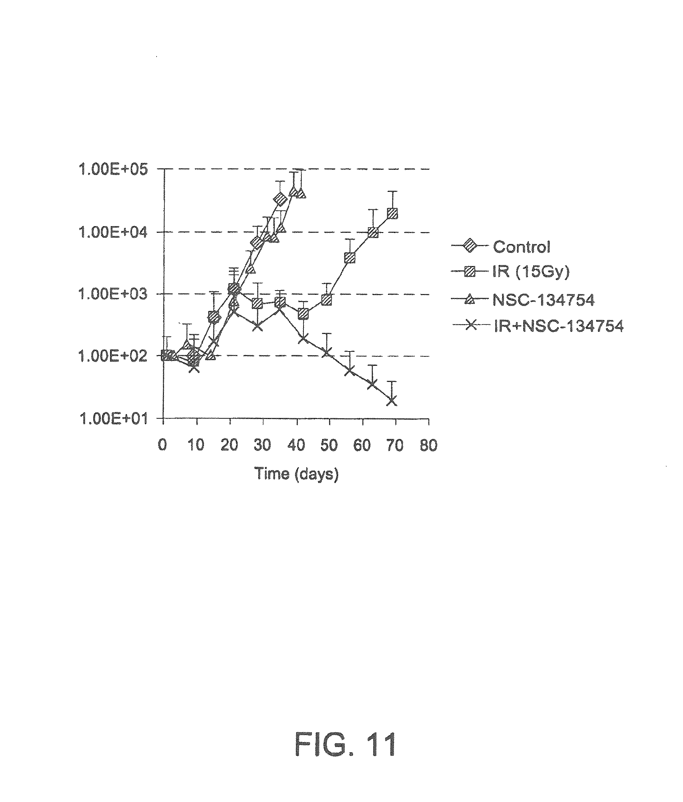

Figure 11 shows the growth of glioblastoma tumors that remained untreated or that were treated with irradiation on day 20 (IR) and/or a HIF-1α inhibitor (NSC134754) daily for three weeks starting on day 20. Symbols and error bars indicate the mean ± SEM for 5 animals per group.

### DETAILED DESCRIPTION

### I. Definitions

The following terms are defined for clarity. Terms not defined should be given their ordinary meaning as used in the art. The use of the singular (*i.e*., "a," "an," etc.) contemplates the plural.

As used herein, the "extracellular matrix" (ECM) refers to the non-cellular component of animal tissues, which typically includes fibrous proteins such as collagen, elastin, or reticulin, link proteins such as fibronectin and laminin, and space filling molecules such as glycosaminoglycans. The ECM may further be mineralised to resist compression (as in bone) or dominated by tension-resisting fibers (as in tendon).

As used herein, a "matrix metalloproteinase" (MMP) refers to a metalloproteinase capable of degrading the extracellular matrix (ECM).

As used herein, a "solid tumor" refers to a mass of neoplastic tissue that does not contain cysts or liquid areas. Solid tumors may be benign or malignant and are exemplified by sarcomas, carcinomas, and lymphomas.

As used herein, "neovasculature" refers to the growth of new blood vessels for supplying the cells of a solid tumor with blood and nutrients.

As used herein, "angiogenesis" refers to the growth of new capillaries from preexisting blood vessels.

As used herein, "vasculogenesis" refers to the differentiation of precursor cells (*i*.*e*., angioblasts) into endothelial cells and the *de novo* formation of a primitive vascular network.

As used herein, "expression" of a protein refers to its translation and optionally its secretion by a cell. Expression of a particular protein by a cell may be indicated by a plus sign ("+") following the name of the protein, for example, MMP-9+ cells are cells that express MMP-9, CD11b+ cells are cells that express CD11b, and MMP-9+/CD11b+ cells are cells that express both MMP-9 and CD11b.

As used herein, "radiation therapy" refers to the use of ionizing radiation to control the growth of neoplastic cells, which are often malignant cells. Radiotherapy may be used for curative treatment or palliative treatment, and includes local treatment of a portion of a body as well as total body irradiation (TBI). Where the radiation therapy causes the substantial destruction of the bone marrow, it may be combined with a subsequent bone marrow transplant to repopulate the bone marrow.

As used herein, "treating" a condition refers to administering a therapeutic substance effective to reduce the symptoms of the condition and/or lessen the severity of the condition.

As used herein, the term "therapeutically effective amount" means the total amount of each active component of the pharmaceutical composition or method that is sufficient to show a meaningful patient benefit upon single or multiple dose administration to a subject, e.g., curing, reducing the severity of, ameliorating one or more symptoms of a disorder, , healing of, increase in rate of healing of such conditions, or in prolonging the survival of the subject beyond that expected in the absence of such treatment. When applied to an individual active ingredient, administered alone, the term refers to that ingredient alone. When applied to a combination, the term refers to combined amounts of the active ingredients that result in the therapeutic effect, whether administered in combination, serially or simultaneously.

As used herein, the terms "antagonist," "inhibitor," and "inhibitory compound" are used interchangeable and refer to an agent that inhibits, prevents, inactivates or otherwise reduces the biological activity of a protein or enzyme. Well-known antagonists include, but are not limited to, small molecules, antibodies (e.g., neutralizing antibodies) and fragments thereof, inhibitory RNAs (e.g., antisense RNA, ribozyme, siRNA, shRNA), etc. Antagonism using an inhibitor of does not necessarily indicate a total elimination of vasculogenisis.

The term "induce", "inhibit", "potentiate", "elevate", "increase", "decrease" or the like, e.g., which denote quantitative differences between two states, refer to at least statistically significant differences between the two states.

The term "small molecule" refers to a synthetic or naturally occurring chemical compound, for instance a peptide or oligonucleotide that may optionally be derivatized, natural product or any other low molecular weight (typically less than about 5 kDalton) organic, bioinorganic or inorganic compound, of either natural or synthetic origin. Such small molecules may be a therapeutically deliverable substance or may be further derivatized to facilitate delivery

As used herein, the term "antibody" refers to a protein comprising at least one, and preferably two, heavy (H) chain variable regions (abbreviated herein as VH), and at least one and preferably two light (L) chain variable regions (abbreviated herein as VL). The VH and VL regions can be further subdivided into regions of hypervariability, termed "complementarity determining regions" ("CDR"), interspersed with regions that are more conserved, termed "framework regions" (FR). The extent of the framework region and CDR's has been precisely defined (see, Kabat, E. A., et al. (1991) Sequences of Proteins of Immunological Interest, Fifth Edition, U.S. Department of Health and Human Services, NIH Publication No. 91-3242, and Chothia, C. et al. (1987) J. Mol. Biol. 196:901-917, which are incorporated herein by reference). Each VH and VL is composed of three CDR's and four FRs, arranged from amino-terminus to carboxy-terminus in the following order: FR1, CDR1, FR2, CDR2, FR3, CDR3, FR4.

The antibody can further include a heavy and light chain constant region, to thereby form a heavy and light immunoglobulin chain, respectively. In one embodiment, the antibody is a tetramer of two heavy immunoglobulin chains and two light immunoglobulin chains, wherein the heavy and light immunoglobulin chains are inter-connected by, e.g., disulfide bonds. The heavy chain constant region is comprised of three domains, CH1, CH2 and CH3. The light chain constant region is comprised of one domain, CL. The variable region of the heavy and light chains contains a binding domain that interacts with an antigen. The constant regions of the antibodies typically mediate the binding of the antibody to host tissues or factors, including various cells of the immune system (e.g., effector cells) and the first component (Clq) of the classical complement system.

As used herein, the term "immunoglobulin" refers to a protein consisting of one or more polypeptides substantially encoded by immunoglobulin genes. The recognized human immunoglobulin genes include the kappa, lambda, alpha (IgA1 and IgA2), gamma (IgG1, IgG2, IgG3, IgG4), delta, cpsilon and mu constant region genes, as well as the myriad immunoglobulin variable region genes. Full-length immunoglobulin "light chains" (about 25 Kd or 214 amino acids) are encoded by a variable region gene at the NH2-terminus (about 110 amino acids) and a kappa or lambda constant region gene at the COOH-terminus. Full-length immunoglobulin "heavy chains" (about 50 Kd or 446 amino acids), are similarly encoded by a variable region gene (about 116 amino acids) and one of the other aforementioned constant region genes, e.g., gamma (encoding about 330 amino acids).

As used herein, "isotype" refers to the antibody class (e.g., IgM or IgG1) that is encoded by heavy chain constant region genes.

The term "antigen-binding fragment" of an antibody (or simply "antibody portion," or "fragment"), as used herein, refers to one or more fragments of a full-length antibody that retain the ability to specifically bind to an antigen (e.g., CD3). Examples of binding fragments encompassed within the term "antigen-binding fragment" of an antibody include (i) a Fab fragment, a monovalent fragment consisting of the VL, VH, CL and CH1 domains; (ii) a F(ab').sub.2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding fragment" of an antibody. These antibody fragments are obtained using conventional techniques known to those with skill in the art, and the fragments are screened for utility in the same manner as are intact antibodies. An antibody or an immunoglobulin chain can be humanized by methods known in the art. Humanized antibodies, including humanized immunoglobulin chains, can be generated by replacing sequences of the Fv variable region which are not directly involved in antigen binding with equivalent sequences from human Fv variable regions. General methods for generating humanized antibodies are provided by Morrison, S. L., 1985, Science 229:1202-1207, by Oi et al., 1986, BioTechniques 4:214, and by Queen et al. U.S. Pat. No. 5,585,089, U.S. Pat. No. 5,693,761 and U.S. Pat. No. 5,693,762, the contents of all of which are hereby incorporated by reference. Those methods include isolating, manipulating, and expressing the nucleic acid sequences that encode all or part of immunoglobulin Fv variable regions from at least one of a heavy or light chain. Sources of such nucleic acid are well known to those skilled in the art and, for example, may be obtained from a hybridoma producing an antibody against a predetermined target. The recombinant DNA encoding the humanized antibody, or fragment thereof, can then be cloned into an appropriate expression vector.

Humanized or CDR-grafted antibody molecules or immunoglobulins can be produced by CDR-grafting or CDR substitution, wherein one, two, or all CDRs of an immunoglobulin chain can be replaced. See e.g., U.S. Pat. No. 5,225,539; Jones et al. 1986 Nature 321:552-525; Verhoeyan et al. 1988 Science 239:1534; Beidler et al. 1988 J. Immunol. 141:4053-4060; Winter U.S. Pat. No. 5,225,539, the contents of all of which are hereby expressly incorporated by reference. Winter describes a CDR-grafting method which may be used to prepare the humanized antibodies (UK Patent Application GB 2188638A, filed on Mar. 26, 1987; Winter U.S. Pat. No. 5,225,539), the contents of which is expressly incorporated by reference. All of the CDR's of a particular human antibody may be replaced with at least a portion of a non-human CDR or only some of the CDRs may be replaced with non-human CDRs. It is only necessary to replace the number of CDRs required for binding of the humanized antibody to a predetermined antigen.

Antibodies may be monoclonal, chimeric and humanized antibodies, which have been modified by, e.g., deleting, adding, or substituting other portions of the antibody, e.g., the constant region. For example, an antibody can be modified as follows: (i) by deleting the constant region; (ii) by replacing the constant region with another constant region, e.g., a constant region meant to increase half-life, stability or affinity of the antibody, or a constant region from another species or antibody class; or (iii) by modifying one or more amino acids in the constant region to alter, for example, the number of glycosylation sites, effector cell function, Fc receptor (FcR) binding, complement fixation, among others.

Antagonists encompass the use of RNA interference ("RNAi"), e.g., against HIF-1α. RNAi can be initiated by introducing nucleic acid molecules, e.g. synthetic short interfering RNAs ("siRNAs") or RNA interfering agents, to inhibit or silence the expression of target genes. See, for example, U.S. Patent Pub. Nos. 2003/0153519 and 2003/01674901, and U.S. Pat. Nos. 6,506,559, and 6,573,099.

An "inhibitory RNA" as used herein is any agent that interferes with or inhibits expression of a target gene or genomic sequence by RNA interference. Such RNA interfering agents include, but are not limited to, nucleic acid molecules including RNA molecules which are homologous to the target gene or genomic sequence, or a fragment thereof, short interfering RNA (siRNA), short hairpin or small hairpin (shRNA), and small molecules which interfere with or inhibit expression of a target gene by RNA interference.

As used herein, "inhibition of target gene expression" includes any decrease in expression or protein activity or level of the target gene or protein encoded by the target gene. The decrease may be of at least 30%, 40%, 50%, 60%, 70%, 80%, 90%, 95%, or 99% or more as compared to the expression of a target gene or the activity or level of the protein encoded by a target gene that has not been targeted by an RNA interfering agent.

An siRNA may be chemically synthesized, may be produced by in vitro transcription, or may be produced within a host cell. Typically, an siRNA is at least 15-50 nucleotides long, e.g., 20,21,22,23,24,25,26,27,28,29, or 30 nucleotides in length. In one embodiment, the siRNA is a double stranded RNA (dsRNA) of about 15 to about 40 nucleotides in length, for example, about 15 to about 28 nucleotides in length, including about 19, 20, 21, or 22 nucleotides in length, and may contain a 3' and/or 5' overhand on each strand having a length of about 0, 1, 2, 3, 4, 5, or 6 nucleotides. In one embodiment, the siRNA can inhibit a target gene by transcriptional silencing. Preferably the siRNA is capable of promoting RNA interference through degradation or specific post-transcriptional gene silencing (PTGS) of the target messenger RNA.

Useful siRNAs include small hairpin RNAs (shRNAs). shRNAs are composed of a short (e.g. about 19 to about 25 nucleotide) antisense strand, followed by a nucleotide loop of about 5 to about 9 nucleotides, and the analogous sense strand. Alternatively, the sense strand may precede the nucleotide loop structure and the antisense strand may follow. These shRNAs may be contained in plasmids and viral vectors.

The targeted region of the siRNA molecules may be selected from a given target sequence. For example, nucleotide sequences can begin from about 25-100 nucleotides downstream of the start codon. Nucleotide sequences can contain 5' or 3' untranslated regions, as well as regions near the start codon. Methods for the design and preparation of siNRA molecules are well known in the art, including a variety of rules for selecting sequences as RNAi reagents (see, e.g., Boese et al., Methods Enzymol. 392:73-96 (2005)).

siRNA may be produced using standard techniques as described in Hannon, (2002) Nature, 418:244-251 (2002); McManus et al., (2002) Nat. Reviews, 3:737-747 (2002); Heasman, (2002) Dev. Biol., 243:209-214 (2002); Stein, (2001) J. Clin. Invest., 108:641-644 (2001); and Zamore, (2001) Nat. Struct. Biol., 8(9):746-750 (2001). Preferred siRNAs are 5-prime phosphorylated.

siRNA inhibitors can be used to target a matrix metalloproteinase, to inactivate bone marrow (BM)-derived cells that express matrix metalloproteinase, and HIF-1α. Antisense oligonucleotides can also be used to matrix metalloproteinase, to inactivate bone marrow (BM)-derived cells that express matrix metalloproteinase, and HIF-1α. "Antisense," as used herein, refers to a nucleic acid capable of hybridizing to a portion of a coding and/or noncoding region of mRNA by virtue of sequence complementarity, thereby interfering with translation from the mRNA. Antisense nucleic acids may be produced using standard techniques as described in Antisense Drug Technology: Principles, Strategies, and Applications, 1st ed., eEd. Crooke, Marcel Dekker (, 2001). The sequence of a BMPRII antisense oligonucleotide is set forth in Vitt et al., Biol. Reprod. 67:473-480 (2002)).

### II. Introduction

Solid tumor growth requires neovasculature to supply the neoplastic cells with nutrients. Neovasculature results from the sprouting of local vessels (angiogenesis) and from the infiltration of circulating bone marrow (BM)-derived cells (vasculogenesis).

Using an animal model to simulates the recurrence of tumors after high dose radiation therapy, it was discovered that matrix metalloproteinase-9 (MMP-9), an enzyme that degrades the extracellular matrix, is required for vasculogenesis in tumors that grow on irradiated tissues or for the regrowth of tumors following irradiation, and that the primary source of MMP-9 in these tumors was BM-derived CD11b-expressing myelomonocytic cells. It was also discovered that BM-derived CD11b-expressing myelomonocytic cells respond to the upregulation of HIF-1α after radiation therapy.

The present compositions and methods relate to inhibiting or reducing the regrowth of solid tumors following radiation therapy by administration of an inhibitor of vasculogenesis that acts by inactivating BM-derived CD11b+/MMP-9+ cells, by inactivating MMP-9, and/or by inactivating HIF-1α.

### III. Method for inhibiting the regrowth of solid tumor following radiation treatment

The present compositions and methods relate to inhibition of solid tumor growth on irradiated tissues, which frequently occurs following radiation therapy to treat a tumor in a human or non-human animal. In particular, the compositions and methods relate to the inhibition of vasculogeneis, e.g., using antagonists of a matrix metalloproteinase (MMP) or bone marrow (BM) cells that express a MMP, as an adjunct therapy following radiation therapy for treating a solid tumor. In particular embodiments, the MMP is MMP-9. In other particular embodiments the bone marrow (BM) cells that express a MMP in the tumors are CD11b+ myelomonocytic cells.

The compositions and methods include inhibiting an MMP to inhibit tumor regrowth by administering to a human or non-human animal a small molecule inhibitor of an MMP, an antibody specific for an MMP, an antisense RNA or other inhibitory RNA specific for mRNAs encoding an MMP, by deleting or disrupting a gene encoding an MMP, or by otherwise causing the inactivation or depletion of an MMP protein or a nucleic acid encoding an MMP protein, in cells of a human or non-human animal. Inhibiting a MMP may also be accomplished by increasing the levels of tissue inhibitors of metalloproteinases (TIMPs) that bind to activated MMPs. Known MMP inhibitors, including MMP-9 inhibitors, include but are not limited to marimastat (BB-2516; CellTech); prinomastat (AG3340; Agouron); BMS-275291 (D2163; Chiroscience); CGS 27023A (Novartis); tanomastat (BAY 12-9566; Bayer Corporation); Trocade (Ro 32-3555; Roche); the tetracycline antibiotics; and the bisphosphonates (as exemplified). Antibodies specific for MMP-9 may be IgG, IgM, IgA, or IgE or a fragment, thereof, such as an Fab fragment. Such antibodies may be polyclonal, monoclonal, synthetic, single chain, chimeric, humanized, CDR-grafted, pegylated or otherwise modified.

The compositions and methods include preventing the infiltration into a tumor of BM-derived cells that express an MMP, such as by killing, sequestering, or otherwise inactivating such cells; thereby preventing the expressing of the MMP at the site of tumor growth or regrowth. Such cells may be inactivated, e.g., by administering to a human or non-human animal a small molecule or an antibody specific to an antigen on the cells. In a particular embodiment, the cells are CD11b+ myelomonocytic cells, and the antibody is directed to CD11b+ or another antigen specific to CD11b+ myelomonocytic cells, such as CXCR4, CD18, or Gr-1, which is present on at least a subset of CD11b+ myelomonocytic cells (*i.e*., CD11b+/Gr-1+ cells). Such antibodies may be IgG, IgM, IgA, or IgE or a fragment, thereof, such as a Fab fragment. Such antibodies may be polyclonal, monoclonal, synthetic, single chain, chimeric, humanized, CDR-grafted, pegylated or otherwise modified, or associated with a cytotoxic agent. In another embodiment, the cells are CD11b+ myelomonocytic cells and small molecule such as AMD3100, which prevents CD11b cells from homing to the tumor vasculature, e.g., by preventing CXCR4 binding to SDF-1.

In another embodiment, BM-derived cells that express an MMP are inactivated using an antagonist to CXCR4, e.g., AMD3100. Other antagonists to CXCR4 that may be used are well-known in the art. Nonlimiting examples include T134, a small analog (14 amino acid residues) ofpolyphemusin II (Arakaki, R et al. (1999) J. Virology 73:1719-1723), and ALX40-4C (Benjamin J. et al AIDS Research and Human Retroviruses. (2001), 17: 475-486). In one embodiment, infiltration into a tumor of BM-derived cells that express an MMP is inhibited or otherwise reduced by antagonizing HIF-1α. Well-known HIF-1α antagonists include, but are not limited to, NSC134754, YC-1 (3-(5'-hydroxymethyl-2'-furyl). 1-benzylindazole )(Yeo et al, (2003) JNCI 95(7):516-525) and Echinomycin (Kong, D et al 2005 (Cancer Res. Oct 1;65:9047-55).

The compositions and methods are applicable to a variety of different type of tumors, e.g., derived from a variety of different type of cells. Tumors derived from head/neck cancer, mouth cancer, and glioblastoma were exemplified and generally behaved similarly in response to inactivation of CD11b+ myelomonocytic cells. Note that the cells of a particular tumor may, in some cases, themselves be a source of a MMP (including MMP-9), which can readily be determined by histological staining. In such cases, it may be preferable to inhibit the MMP directly, rather than to kill, sequester, or otherwise inactivate the cells expressing the MMP.

An inhibitor of vasculogenesis may also be administered in combination with an additional therapeutic agent for killing tumor cells, reducing metastasis, or another purpose. One or more MMP inhibitors and/or inhibitors of a cell expressing MMPs may be administered.

An inhibitor of vasculogenesis may be delivered in a suitable pharmaceutical vehicle. The vehicle may be selected for intravenous or intraarterial administration, and may include a sterile aqueous or non-aqueous solution that may include preservatives, bacteriostats, buffers and antioxidants known to the art. In the aerosol form, the inhibitor may be used as a powder, with properties including particle size, morphology and surface energy known to the art for optimal dispersability. In tablet form, a solid vehicle may include, for example, lactose, starch, carboxymethyl cellulose, dextrin, calcium phosphate, calcium carbonate, synthetic or natural calcium allocate, magnesium oxide, dry aluminum hydroxide, magnesium stearate, sodium bicarbonate, dry yeast or a combination thereof. The tablet preferably includes one or more agents which aid in oral dissolution. The inhibitors may also be administered in forms in which other similar drugs known in the art are administered, including patches, a bolus, time release formulations, and the like.

An inhibitor of vasculogenesis may be administered to a patient by a variety of routes. For example, the inhibitors may be administered parenterally, including intraperitoneally; intravenously; intraarterially; subcutaneously; intramuscularly; or intratumorally. The inhibitors may be administered locally to tissues at the site of a tumor or surgically implanted underneath the skin. The inhibitors may also be administered via a mucosal surface, including rectally, and intravaginally; intranasally; by inhalation, either orally or intranasally; orally, including sublingually; intraocularly and transdermally. Combinations of these routes of administration are also envisioned.

Suitable carriers, diluents and excipients are well known in the art and include materials such as carbohydrates, waxes, water soluble and/or swellable polymers, hydrophilic or hydrophobic materials, gelatin, oils, solvents, water, and the like. The particular carrier, diluent or excipient used will depend upon the means and purpose for which the compound is being applied. In general, safe solvents are non-toxic aqueous solvents such as water and other non-toxic solvents that are soluble or miscible in water. Suitable aqueous solvents include water, ethanol, propylene glycol, polyethylene glycols (e.g., PEG400, PEG300), etc. and mixtures thereof. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents and other known additives to provide an elegant presentation of the drug or aid in the manufacturing of the pharmaceutical product (i.e., medicament). Some formulations may include carriers such as liposomes. Liposomal preparations include, but are not limited to, cytofectins, multilamellar vesicles and unilamellar vesicles. Excipients and formulations for parenteral and nonparenteral drug delivery are set forth in Remington, The Science and Practice of Pharmacy (2000).

The skilled artisan will be able to determine the therapeutically effective or optimum dosage and frequency of administration based on pharmacokinetic data relating to the particular inhibitor and its target molecules, such as the chemical nature of the particular inhibitor (e.g., soluble small molecule, antibody, pegylated antibody, etc.), the route of delivery (e.g., oral, intratumoral/local, systemic), and the particular formulation selected, (e.g., slow release-oral, pegylated-antibody or small molecule, etc.).

In some embodiments, an inhibitor of is administered to a human or non-human animal (*i.e.*, subject) following radiation treatment to kill tumor cells at a tumor site. The radiation treatment may be localized to a portion of the subject or may involve the entire subject and may be a single dose or series or doses separated by any amount of time. An inhibitor of vasculogenesis may also be administered prior to radiation treatment and continued following radiation treatment, or administered during the course of a series of radiation treatments.

The following examples are intended to illustrate the present compositions and methods, or the experiments leading thereto, and should not be considered limiting in scope.

### EXAMPLES

### Experimental Procedures

### Animals

All mice except C3H (FVB/N-TgN(*TIE2*-lacZ)182Sato; B6.Cg-Tg(*TIE2*GFP)287Sato/1J; B6;129S-Gt(ROSA)26Sor/J; FVB-Tg(ITGAM-DTR/EGFP)34Lan/J; FVB.Cg-Tg(CFPU)5Nagy/J; FVB.Cg-*Mmp9*^{tm1Tvu}/J; FVBINJ; C57B1/6J; and athymic nu/nu nude mice were purchased from the Jackson laboratory (Bar Harbor, ME). C3H mice were obtained from the breeding facility at Stanford University's Research Animal Facility. For the glioblastoma multiforme experiments athymic nu/nu nude mice were purchased from Charles River (Wilmington, MA). Transgenic green fluorescent protein-expressing nude mice (GFP-nude mice) were obtained from AntiCancer Inc. (San Diego, CA). Mice were maintained in a germ-free environment and had access to food and water available *ad libitum.*

### Bone marrow transplantation

Six- to twelve-week-old mice were used as (bone marrow) BM recipients and donors. BM cells from the donors were harvested from both femurs and tibias by flushing the bone cavity with Hank's balanced salt solution (Invitrogen, Carlsbad, CA) using 25 gauge needles (BD, Franklin Lakes, NJ). The recipient mice were lethally irradiated 24 hr prior to the BM transplantation. The IR doses used were 9 Gy for FVB, *MMP-9* KO, and C3H mice, and 9.5 Gy for C57B1/6 mice. The lethally irradiated mice received intravenously a suspension of greater than 2×10⁶ BM cells and were allowed to recover for at least 4 weeks. For glioblastoma studies, ten- to twelve-week-old GFP nude mice were used as bone marrow (BM) recipients and donors. The IR dose used was 8.5 Gy. The lethally irradiated mice received intravenously a suspension of greater that 3 x 10⁶ BM cells and were allowed to recover for at least 4 weeks.

### Cell lines

MT1A2 mouse mammary carcinoma cells were obtained from Dr. Frank Graham (McMaster University, Canada). TG1-1 mouse mammary carcinoma cells were obtained from Dr. Rakish Jain (Harvard University, MA). 6780 lymphoma cells were obtained from Dr. Dean Felsher (Stanford University, CA). B16F1 melanoma cells were obtained from Dr. Garth Nicolson (UC Irvine, CA). LLC cells were purchased from the American Tissue Culture Collection (ATCC; Manassas, VA).

U251 human glioblastoma multiforme (GBM) cells were obtained from Dr. RK Puri (Food and Drug Administration, Bethesda, MD). Murine transformed HIF-1 wild type or knock out GBM cells were obtained from Dr. G Bergers (University of California San Francisco, San Francisco, CA). The GBM cells were transduced with firefly Luciferase gene, HIF-1 promoter region HRE-containing Luciferase gene, or short interfering RNA for HIF-1 knock down by non-replicative retrovirus.

MT1A2, TG1-1, RIF, BI6F1, LLC, and GBM cells were maintained in Dulbecco's modified Eagle's medium (DME; Invitrogen, Carlsbad, CA) supplemented with 10 % fetal bovine serum (FBS, Mediatech, Inc., Herndon, VA), and penicillin-streptomycin (1%). 6780 cells were grown in RPMI medium (Invitrogen) supplemented with 10 % FBS, and penicillin-streptomycin (1%). RIF cells were constantly passaged *in vitro-in vivo* by implanting in syngeneic female C3H mice as described previously (Twentyman *et al.,* 1980). *Tumor implantation, irradiation, measurement, and perfusion*

The MT1A2 and TGI-1 cells (1.5×10⁶ cells/mouse), 6780 cells (5×10⁶ cells/mouse), or RIF, B16F1, or LLC cells (5×10⁵ cells/mouse) were inoculated intradermally on the backs of mice approximately 1 cm proximal to the base of the tail. Unanaesthetized mice were placed in lead jigs, through which the tumor implantation site (approximately 200 mm³ in volume) protruded for irradiation. For glioblastoma studies, U251 (1×10⁶ cells/mouse) or murine GBM (1 X 10⁵ cells/mouse) were inoculated intracranially on the brain exactly 2mm to the right of the midline and I mm anterior from the bregma. Anaesthetized mice were placed in lead jigs, with a cut out which allowed which whole brain irradiation including the tumor. The mouth and throat were covered and not exposed to irradiation.

Irradiation was performed with a Phillips X-ray unit operated at 200 kVp with the dose rate of 1.21 Gy/min (20 mÅ with added filtration of 0.5 mm copper, the distance from X-ray source to the target of 31 cm and a half value layer of 1.3 mm copper). Where tumors were grown in pre-irradiated sites (*i.e*., an irradiated bed) the tumors were implanted 5 days following irradiation of the sites.

Tumor volume (*V*) was calculated using the formula for a spheroid: *V* = π/6 × (width)² × (length). When tumor volumes reached approximately 200 mm³ for control tumors and slightly more than 200 mm³ for irradiated tumors and tumors grown in the irradiated bed, cardiac perfusion was performed in asphyxiated tumor bearing mice with 4% paraformaldehyde in phosphate buffered saline (PBS; Invitrogen). Tumors were removed, embedded in optimal cutting temperature (OCT) compound (Sakura Finetek, Torrance, CA), and frozen in -80°C until cryosectioning and immunostaining.

Tumor growth was analyzed by using an IVIS in vivo imaging system (Caliper Life Sciences, Hopkinton, MA) to monitor the bioluminescence imaging of brain tumor. When tumor bioluminescent signal reached approximately 50 times increased compared to the data of the day 1, cardiac perfusion was performed in asphyxiated tumor bearing mice with 4% paraformaldehyde in phosphate buffered saline (PBS; Invitrogen). Mice brains including tumors were removed, embedded in optimal cutting temperature (OCT) compound (Sakura Finetek, Torrance, CA) and frozen in -80°C until cryosectioning and immunostaining.

### Immunostaining

Primary antibodies for immunofluorescent staining included a rat monoclonal CD31/PECAM-1 (MEC13.3; BD Pharmingen, San Diego, CA), a rabbit polyclonal GFP antibody (Invitrogen, Eugene, OR), a rabbit polyclonal MMP-9 (Abcam, Cambridge, MA), a biotinylated CD11b/macrophage-associated antigen-1α (M1/70; BD Pharmingen), a biotinylated CD11c (HL3; BD Pharmingen), a biotinylated Gr-1/Ly-6G (RB6-8C5; BD Pharmingen), rat monoclonal F4/80 (A3-1, Gnetex, Irvine, CA), and CD45 (BD Pharmingen). Anti phosphorylated CXCR-4 polyclonal antibody was obtained from Dr. JB Rubbin at Washington University.

Primary antibodies were detected by using secondary antibodies of anti-rat AlexaFluor 594 (Invitrogen), anti-rabbit AlexaFluor 488 (Invitrogen), anti-rabbit AlexaFluor 555 (Invitrogen), anti-rabbit AlexaFluor 647 (Invitrogen), streptavidin AlexaFluor 488 (Invitrogen) or streptavidin AlexaFluor 555 (Invitrogen). FITC-conjugated anti-mouse alpha-smooth muscle actin (α-SMA) antibody (Sigma, St. Louis, MO) was used to detect pericytes, and Phycoerythrin (PE) -conjugated antibodies for CD4 (StemCell Technologies, Vancouver, BC, Canada), CD8α/Ly-2 (53-6.7; BD Pharmingen), and CD49b (StemCell Technologies) were used to detect CD4, CD8α T cells, and NK cells, respectively.

Unless otherwise indicated, 8 µm frozen sections of tumors were dried in air, hydrated with PBS, blocked with 5 % goat serum in PBS (containing 0.03 % Triton X-100) for 30 min, and incubated with primary antibodies for 2 hr at room temperature (RT). Sections were washed three times in PBS, followed by secondary antibody for 1 hr at RT. For glioblastomas, 5 µm frozen sections of tumors were dried in air, hydrated with PBS, blocked with 5 % goat serum in PBS (containing 0.03% Triton X-100) for 30 min, and incubated with primary antibodies over night at 4°C. Sections were washed three times in PBS, followed by secondary antibody for 40 min at room temperature.

After washing in PBS, sections were mounted with anti-fade reagent with 4',6-diamidino-2-phenylindole (DAPI) (Invitrogen) and viewed with Leica DMRA2 microscope (Wetzlar, Germany) using Plan 20×/0.40 and 40×/0.65 objective lenses with HC PLAN s 10×/22 eyepieces. Images were acquired with a Hamamatsu ORCA-ER camera and Improvision OpenLab software.

### Histological assessment

MMP-9 positive area in the tumors was determined by the point-count method (Gray, 1996). Briefly, the proportions were calculated as the number of points directly over MMP-9 positive staining divided by the total number of points examined using a six-point grid in a 15 × eyepieces at a 10 × objective.

Quantitative analysis for CD11b and MMP-9 positive cells was done by counting the number of cells in the photographed fields where the most CD11b were observed in non-necrotic regions using a 40 × objective with 10 × eyepieces of the DMRA2 fluorescent microscope. X-gal positive cells were counted in 5 random fields per tumor with a 20 × objective and 10 × eyepieces of the DMLB microscope. Quantification was made on 3-5 independent specimens per tumor, 4-5 animals per group.

GFP positive area in glioblastoma tumors was determined by the point-count method (Gray, 1996). Briefly, the proportions were calculated as the number of points directly over GFP positive staining divided by the total number of points examined using a six-point grid in a 15 x eyepieces at a 10 x objective.

Quantitative analysis for GFP/PcpCXCR4 and GFP/CD11b positive cells was done by counting the number of cells in the photographed fields where five to ten fields were randomly selected in non-necrotic regions using a 40 x objective with 10 x eyepieces of the DMRA2 fluorescent microscope. Quantification was made on 3 independent specimens per tumor, 3-5 animals per group.

### Chemotaxis and Attachment Assessment

Whole bone marrow cells were isolated from tibia and femur of a nude mouse. Red blood cells were lysed using PharMLysis buffer followed by washing with phosphate buffer solution containing 2 % fetal bovine serum. The bone marrow cells were subjected to density gradient separation followed by labeling with CFSE. Labeled cells were counted and 1 X 10⁵ cells were introduced used for either the chemotaxis or attachment assay.

For the chemotaxis assay, cells were introduced to the upper compartment of a transwell chamber separated by media with or without chemokines, e.g., 10 % fetal bovine serum, 0.1 µg/ml VEGF or 0.1 µg/ml M-CSF. After 4 hr of incubation 37° C, the upper chamber was removed and fixed with 4 % paraformaldehyde. The upper membrane was rigorously wiped using a cotton swab. The membrane was carefully cut and placed on a glass slide followed by microscopic exam. Green fluorescent (CFSE) labeled cells were counted at 20X lens.

For the attachment assay, cells were introduced to a monolayer of C166 endothelial cells grown in glass chamber slide. The bone marrow cells were allowed to attach for 1 hr at 37°C, after which unattached cells were removed by washing with phosphate buffer solution. The attached cells were fixed with 4% paraformaldehyde. The chambers were removed and the slides were examined microscopically.

### Drug treatment

ZA (Zometa; Novartis Pharma AG) was dissolved in sterile water and stored long term at -80 °C and at 4 °C for short-term storage as reported previously (Oiraudo *et al.,* 2004). Mice were treated every day with ZA or water from the first day of the local irradiation at the lower back. The animals were monitored during the treatment for their body weight to assess side effects and did not show any significant loss in weight (less than 10 % of body weight).

DT (List biological laboratories Inc., CA) was prepared in sterile water containing 1 % of bovine serum albumin (BSA, Sigma). Carrageenan (Sigma) was dissolved in saline at 10 mg/ml. Mice were treated with DT or vehicle (1 % BSA in water) once in two days or carrageenan once per week by intraperitoneal injections from the first day of the local irradiation. The treated animals received water containing antibiotics (neomycin and polymyxin B) throughout the study.

NSC-134574 (Developmental Therapeutics Program, NCI, Bethesda, MD) was dissolved in sterile saline and stored at -20°C. Mice were treated daily with NSC-134754 or vehicle (saline) for three weeks following irradiation. The animals were monitored during the treatment for their body weight to assess side effects and did not show any significant loss in the weight.

AMD3100 (Sigma-Aldrich) was prepared in sterile saline. Mice were treated with AMD3100 or vehicle (saline) for three weeks by osmotic infusion pumps (Durect, Cupertino, CA) following irradiation. The animals were monitored during the treatment for their body weight to assess side effects and did not show any significant loss in the weight.

Carrageenan (Sigma) was dissolved in saline at 20 mg/ml at 55°C water bass, and mice were pre-treated with carrageenan 1, 3, 7 days prior to tumor inoculation. After tumor inoculation on brain, carragenan treatment was done every five days. The animals were monitored during the treatment for their body weight to assess side effects and did not show any significant loss in the weight.

### Statistical analysis

Statistical comparisons of data sets were performed by a two-tailed Student's *t*-test or one-way ANOVA with Tukey post test (V4.00 GraphPad Inc., CA). The data were considered to be significantly different when probability values of *P* < 0.05.

### Experimental Results

### A. Overview

Animals with different genetic backgrounds and in some cases lethally irradiated and transplanted with bone marrow (BM) from a genetically identical or different animal were used to determine the mechanism by which solid tumors regrow following radiation treatment. BM-derived cells present in a tumor could be distinguished from other cells in the tumor by the expression of green fluorescent protein (GFP), which was constitutively expressed in the cells of the transplant donor. The methods used in the experiments are described in the appended Examples.

The experiments required that the tissues of the tumor transplantation site in the animals receive a dose of radiation (20 Gy) sufficient to sterilize/kill essentially all the endothelial cells in the tissue prior to tumor transplantation and hence abrogate local angiogenesis (Udagawa et al. (2007) Cancer Res. 67:2040-45.). By abrogating local angiogenesis, any vasculogenesis observed in the tumors resulted from the infiltration of endothelial progenitor cells (EPCs) derived from the BM.

The experiments demonstrate the role played by BM-derived CD11b+ myelomonocytic cells expressing MMP-9 in remodeling the extracellular matrix (ECM) to promote tumor vasculogenesis in irradiated tissues. MMP-9 appears to mediate degradation of the ECM allowing endothelial cells to migrate to a tumor site when existing endothelial cells in (and adjacent to) the tumor are unable to proliferate due to local irradiation.

### B. CD11b+ myelomonocytic cells are present in tumor infiltrates

Infiltrates from tumors grown on BM-recipient animals were examined to determine the cells responsible for tumor regrowth. The infiltrates were from MT1A2 tumors grown in pre-irradiated tissues on the backs of mice that had received BM cells from mice ubiquitously expressing green fluorescent protein (GFP). GFP thereby served as a marker for cells of BM-origin. These cells were examined by double-label immunofluorescent staining immunostaining for the presence of markers characteristic of inflammatory cells, including cytotoxic T-cells (CD8α), helper T-cells (CD4), natural killer cells (CD49b), monocytes/macrophages (CD11b), dendritic cells (CD11c), and granulocytes/neutrophils (Gr-1). A significant number of GFP-expressing (GFP+) cells co-localized with CD11b-expressing (CD11b+) cells (data not shown). CD4 and CD8α expressing cells were also detected in the tumors but did not co-localized with GFP expressing cells (data not shown), suggesting that these cells were not derived from the BM but possibly from the thymus or spleen. CD11c, Gr-1, or CD49b expressing cells that also expressed GFP were not observed (data not shown). These results suggested that BM-derived CD11b+ myelomonocytic cells were associated with tumor regrowth.

To determine whether the BM-derived CD11b+ myelomonocytic cells infiltrating tumors were affected by irradiation, tumors were grown either (i) on non-irradiated tissues (control), (ii) on pre-irradiated tissues (pre-IR bed), as before, (iii) or on non-irradiated tissues until established, irradiated with a single dose (20 Gy) of radiation, and allowed to regrow beyond the volume at which they were irradiated (IR tumor). Immunostaining demonstrated that infiltrates obtained from the MT1A2 tumors of pre-irradiated tissues, and tissues irradiated after tumor established followed by regrowth of the tumor, contained significantly more CD11b+ cells compared to control MT1A2 tumors of the same size grown on non-irradiated tissues (Figures 1).

These results demonstrated that CD11b+ myelomonocytic cells are present in the BM-derived EPCs isolated from tumor cells grown on irradiated tissues.

### C. CD11b+ myelomonocytic cells express MMP-9

Since monocytes and macrophages are known to promote tumor angiogenesis (Coussens et al. (2000) Cell 103:481-90; Giraudo et al. (2004) J. Clin. Invest. 114:623-633; Lin et al. (2006) Cancer Res. 66:11238-46), experiments were performed to determine whether CD11b+ myelomonocytic cells are involved with vasculogenesis, e.g, by remodeling the extracellular matrix (ECM) in the tumors grown on irradiated tissues.

Double-label immunofluorescent histological staining of tumor tissue was performed using one antibody specific for CD11b+ and another antibody specific for matrix metaloproteinase-9 (MMP-9), which is known to be involved with ECM remodeling The results of the staining demonstrated that most of the CD11b+ cells were also MMP-9+ (data not shown) and that irradiated tumors (IR tumors) and tumors grown in the irradiated bed (pre-IR bed) showed an increased number of CD11b/MMP-9 double-positive cells (Figure 2A). Immunoblot analysis was performed to confirm the histological results. As before, irradiated tumors (IR tumors) and tumors grown in the irradiated bed (pre-IR bed) showed increased expression of MMP-9 (Figure 2C).

### D. Depletion of MMP-9 abrogates tumor vasculogenesis

To address the role of MMP-9 in vasculogenesis, *MMP-9* knockout (KO) mice were used in experiments similar to those described above. MT1A2 tumors grew on non-irradiated *MMP-9* KO mice although more slowly than on non-irradiated "WT" mice, as used above (Figure 3E). However, tumor growth on irradiated tissues was severely impaired in *MMP-9* KO mice compared to WT mice (Figures 3A and 3F). Tumor growth on pre-irradiated tissues was severely impaired in *MMP-9* KO that were lethally irradiated and then received a transplant of BM cells from the same *MMP-9* KO mice (*MMP-9* KO mice + KO BM) (Figure 3A).

To determine whether functional BM could restore tumor growth in pre-irradiated tissues of *MMP-9* KO mice, *MMP-9* KO mice were lethally irradiated and then received a transplant of BM cells from either WT mice (*i.e. MMP-9* KO + WT BM mice) or *MMP-9* KO mice as a control (*i.e*. *MMP-9* KO + KO BM mice). The efficiency of BM reconstitution was confirmed by zymography at the time of tumor implantation, which was at least 4 weeks following-BM transplantation (not shown).

Reconstitution (*i.e.,* transplantation) of the BM in irradiated *MMP-9* KO mice with BM from WT mice completely restored the growth of the tumors in the pre-irradiated site to that of WT mice (Figure 3A). Notably, WT mice receiving BM cells from *MMP-9* KO mice (WT mice + KO BM) showed no difference in tumor growth in pre-irradiated tissues compared to WT mice + WT BM (Figure 3A), suggesting that non-BM-derived cells in WT mice can compensate for the lack of MMP-9 in BM cells. This experiment illustrates the benefit of using *MMP-9* KO to dissect the mechanism behind tumor regrowth.

While there was no significant difference in the number of CD11b+ cells in the tumors from the four different lethally irradiated, BM-transplanted mice (*i.e., MMP-9* KO + WT BM, *MMP-9* KO + KO BM, WT + WT BM, and WT + KO BM; Figure 3C), double-labeling experiments demonstrated differences in the number of cells expressing both CD11b+ and MMP-9 that were present in the tumors. Ixnmunostaining showed that in tumors grown on pre-irradiated tissues of *MMP-9* KO + WT BM mice, the majority (91±3%) of MMP-9+ cells were also CD11b+, while there were no CD11b+/MMP-9+ myelomonocytic cells detected in the tumors grown on *MMP-9* KO + KO BM mice or WT + KO BM mice (Figure 3D). The tumors from WT + KO BM mice contained some MMP-9+ cells but with morphology of smooth muscle or fibroblast-like cells (not shown). Overall, these results suggest that CD11b+ myelomonocytic cell s are the primary source of MMP-9 in tumors grown on irradiated tissues, and that these cells and/or MMP-9 promotes tumor growth in irradiated tissues.

To determine whether BM-derived myelomonocytic cells affect tumor regrowth though vasculogenesis, the ability CD11b+ myelomonocytic cells to differentiate into endothelial cells in tumors was examined by immunostaining using antibodies specific for endothelial cells markers. CD11b+ cells did not co-localize with CD31+ cells in the tumors from any of the above four groups of animals (not shown), suggesting that CD11b+ myelomonocytic cells do not differentiate into tumor endothelium cells.

The proportion of CD31+ cells surrounded by α-smooth muscle actin (α-SMA)-expressing pericytes was examined to assess the vessel density and maturity in the tumors. Immunostaining showed that in tumors grown on non-irradiated tissues, most of the vessels in the tumors grown in WT (87± 2 %) or *MMP-9* KO (87± 2 %) mice were associated with α-SMA (Figure 4A), while the vasculature of the tumors growing on the pre-irradiated tissues of WT + WT BM showed minimal expression of α-SMA (Figure B). These observations suggested that the vessels arising in the tumors grown on irradiated vs. non-irradiated tissues had different etiologies; in particular, vessels arising in tumors grown on non-irradiated tissues result from angiogenesis (*i.e.,* local sprouting), while vessels arising in tumors grown on the irradiated tissues only arise from vasculogenesis (growth from circulating cells). Notabaly, the majority of the vessels in the very small (and non-growing) tumors grown on the irradiated tissues of *MMP-9* KO + KO BM mice had a mature appearance with extensive α-SMA expression (Figure 4B), which was presumably the result of angiogenesis in the absence of vasculogenesis.

As shown in Figure 4A, there was little or no difference in the density and maturity of the CD31+ endothelial cells in tumors grown on non-irradiated tissues of WT and *MMP-9* KO mice. Vessel density was significantly lower in the very small non-growing tumors growing in the irradiated site of the *MMP-9* KO mice but was restored to WT levels by transplantation of WT BM cells (Figure 4B).

To determine whether impaired tumor growth in the pre-irradiated tissues of *MMP-*9 KO mice was due to impairment in tumor vessel function, tumors were grown on pre-irradiated tissues of WT, *MMP-9* KO, or *MMP-9* KO + WT BM mice, which were intravenously injected with Hoechst 33342, a fluorescent stain used as an *in vivo* marker for studying functional tumor vasculature. Perfusion of the vasculature was similar in WT, *MMP-9* KO, and *MMP-9* KO + WT BM mice (data not shown).

These results suggested that the failure of the tumors to grow on the pre-irradiated tissues of *MMP-9* KO mice was the result of abrogation of both angiogenesis (by irradiation) and of vasculogenesis (by lack of MMP-9 from BM-derived cells). The results further suggested that WT BM restored tumor growth on irradiated tissues by promoting immature vessel formation by MMP-9+ expressed by CD11b+ myelomonocytic cells via vasculogenesis.

### E. Pharmacological inhibition of CD11b+ /MMP-9+ cells reduces tumor growth

To determine whether selective inhibition of CD11b+ myelomonocytic cells expressing MMP-9 would inhibit BM-derived vasculogenesis and tumor growth in pre-irradiated tissues, several pharmacological/chemical agents were used to inactive CD11b+ myelomonocytic cells.

### 1. Aminobisphosphonate zolendronic acid

In a first set of experiments, aminobisphospbonate zolendronic acid (ZA, Zometa^{®}), a pharmaceutical agent used clinically to ameliorate bone metastases and recently reported to selectively target MMP-9 expressing macrophages in K14-HPV16 cervical carcinoma in mice (Giraudo *et al.,* 2004), was used to inhibit CD11b+ myelomonocytic cells expressing MMP-9. MT1A2 tumors were grown in the pre-irradiated tissues in *MMP-9* KO mice that had received BM cells from WT mice (*MMP-9* KO + WT BM) as previously described. Based on the above experiments, the primary source of MMP-9 in these mice is from the BM-derived CD11b+ myelomonocytic cells.

For comparison and to determine the specific activity of ZA in targeting MMP-9 from BM-derived cells as opposed to from other tissues, ZA was also administered to WT mice receiving BM cells from *MMP-9* KO mice (WT + KO BM). Because such mice lack MMP-9-expressing CD11b+ myelomonocytic cells, the tumor growth on pre-irradiated tissues of these mice should not be affected by the ZA treatment.

Treatment with ZA at 100 µg/kg intraperitoneally once per day for up to 6 weeks produced a significant inhibition of tumor growth on the irradiated site in *MMP-9* KO + WT BM mice (Figure 5A) but had no effect on tumor growth in WT + KO BM mice (Figure 5B). ZA-treated tumors in *MMP-9* KO + WT BM mice had similar numbers of CD11b+ myelomonocytic cells but a smaller fraction of the cells were MMP-9+ (Figure 5C). ZA-treated tumors showed significantly fewer numbers of CD31-expressing vessels than control tumors (Figure 5C).

When examined for vessel maturity, ZA-treated tumors had significantly more vessels associated with α-SMA than the control tumors (Figure 5C) in agreement with the results obtained with *MMP-9* KO mice + KO BM (Figure 4). CD11b+MMP-9+ myelomonocytic cells were not observed in tumor grown on the tissues of WT mice + KO BM mice (not shown, and there was no significant difference in the number of CD11b+ cells in ZA-treated and control tumors (Figure 5D). As above, the percentage of CD31+ tumor vessels associated with α-SMA was lower in the WT + KO BM mice and was not affected by ZA (Figure 5D).

These results demonstrated that ZA efficiently targeted MMP-9 expressed by the BM-derived CD11b+ myelomonocytic cells in the tumor, thereby inhibiting the growth of the tumors on the pre-irradiated tissues and reducing the numbers of immature vessels in the tumors arising through vasculogenesis.

### 2. Neutralizing CD11b antibodies

In a second set of experiments, mice were implanted with a FaDu (mouth cancer) cells and irradiated with 20 Gy (Figure 7A, 7C) or 12 Gy (Figure 7B, 7C), while receiving a neutralizing antibody specific for CD11b, a neutralizing antibody for Gr-1, or a control antibody. As shown in the graphs, the administration of a neutralizing CD11b antibody, but not the administration of a neutralizing antibody for Gr-1 inhibited the regrowth of the FaDu tumor cells following irradiation.

In vitro experiments suggest that neutralizing antibody to CD11b may mediate its post-irradation anti-tumor effects by reducing attachment of CD11b+ cells to the extracellular matrix (Figure 8A) and/or by preventing chemotaxis of CD11b+ cells, e.g., in response to serum (Figure 8C), VEGF (Figure 8D), and M-CSF (Figure 8E). The neutralizing antibody did not have an effect on untreated cells (Figure 8G).

### 3. The CXCR4/SDF-1 bicyclam inhibitor AMD 3100

In a third set of experiments, mice were implanted with a glioblastoma tumor in an early or late tumor model in which the animals were irradiated five times with 2(arrows) on day 11 or with 15 Gy on day 21, respectively, while receiving the CXCR4/SDF-1 bicyclam inhibitor AMD 3100 for three weeks starting on the first day of irradiation (Figure 9). CXCR4 is a receptor for SDF-1 expressed on the surface of CD11b cells. AMD 3100 is a small molecule that prevents CD11b cells from homing to the tumor vasculature. As shown in the Figures 9A and 9B, AMD 3100 significantly inhibited the growth of irradiated tumors but not non-irradiated tumors in both early and late tumor models.

### 4. Carrageenan

In a fourth set of experiments, CD11b+ monocytes were inactivated by treating mice with carrageenan 1, 3, and 7 days prior to implantation of glioblastoma tumr0s. After tumor inoculation, carrageenan treatment was provided every five days. As shown in Figure 10, carrageenan significantly inhibited the growth of irradiated tumors.

The above results demonstrated that depleting, blocking the function of, or otherwise inactivating CD11b+ myelomonocytic cells, e.g., using an aminobisphosphonate, an antibody specific for CD11b, or an antibody specific for CXCR4 (which is present on CD11b+ myelomonocytic cells), all result in decreased tumor regrowth on irradiated tissues. These results demonstrate that different pharmacological agents can be used to inactivate CD11b+ myelomonocytic cells, and that such inactivation leads to inhibition of the regrowth of a variety of different tumor types.

### F. Genetic inactivation of CD11b+ myelomonocytic cells

BM cells from transgenic mice expressing the human diphtheria toxin receptor (DTR) and GFP under the control of *CD11b* promoter (Stoneman *et al.* (2007) Circ. Res. *100*:884-93) were transplanted as above into lethally irradiated *MMP-9* KO mice (*MMP-9* KO mice + DTR BM). Administration of diphtheria toxin (DT; 10 ng/g) effectively depleted GFP positive CD11b+ cells in the peripheral blood in 24 hours (Figure 6A). In contrast, administration of DT to WT mice has no effect on depletion of CD11b+ myelomonocytic cells (Figures 6A and 6B). Repeated administration of DT (*i.e.,* 10 ng/g once in every two days) to *MMP-9* KO + DTR BM mice resulted in mortality in only 20 days (not shown); therefore, the dose was reduced to 5 ng/g DT once every two days, which still depleted the CD11b+ myelomonocytic cell population in the peripheral blood of *MMP-9* KO + DTR BM mice (Figures 6B and 6C), while prolonging survival.

The growth of tumors implanted on the pre-irradiated tissues of *MMP-9* KO + DTR BM mice was significantly inhibited by DT (5 ng/g), similar to the lack of growth in *MMP-9* KO mice (Figure 6D). Treatment of WT + WT BM mice with DT produced no effect on tumor growth (Figure 6E).

These results demonstrate that genetic inactivation of CD11b+ myelomonocytic cells, in additional to pharmacological inactivation, leads to reduced growth following irradiation or on irradiated tissues.

### G. Results with radiation-induced fibrosarcoma (RIF) tumors in C3H mice

The above studies have largely focused on MT1A2 mouse mammary carcinoma in FVB mice; however, experiments were also performed with radiation-induced fibrosarcoma (RIF) tumors in C3H mice. As with the MT1A2 tumors, and increase in MMP-9 expression was observed in irradiated tumors and tumor implanted on irradiated tissues and increased numbers of CD11b+ myelomonocytic cells and MMP-9 positive cells were found in irradiated tumors. However, some RIF tumors were characterized by the presence of few CD11b+ myelomonocytic cells, while still staining for MMP-9 (not shown).

These results suggest that some tumors, such as RIF tumors, are themselves are a significant source of MMP-9. In such tumors, inhibition of MMP-9 directly, rather than inhibition of CD11b+ myelomonocytic cells that produce MMP-9, is likely to be more effective in inhibiting tumor regrowth.

### H. Pharmacological inhibition of HIF-1α

Since postirradiation vasculogeneis is initiated by tumor hypoxia and the upregulation of HIF-1, experiments were performed to determine whether bone marrow derived celis involved with vasculogenesis, e.g., in response to HM-1. It was determined that irradiation of U251 glioblastoma tumors activated HIF-1α and that HIF-1α activity correlated with tumor growth (data not shown). Additionally, it was demonstrated that shRNA gene inactivation of HIF-1α in U251 cells inhibited the ability of the tumors to grow post-irradiation (data not shown).

To determine the effect of HIF-1 α on influx of BM-derived myelomonocytic cells into glioblastoma tumors, brain samples from untreated mice or mice treated with irradiation or irradiation plus a HIF-1 α inhibitor (NSC14134754) were harvested 18 days after irradiation and examined by immunostaining with DAPI and antibodies specific for CD45, CD11b, or F4/80. CD45+CD11b+ and CD45+F4/80+ cells were present in brain samples of animals treated with irradiation, but not control animals or animals treated with irradiation and NSC14134754. The absence of CD45+CD11b+ and CD45+F4/80+ cells in glioblastoma tumors treated with irradiation and NSC14134754 correlated with decreased post-irradiation tumor growth (Figure 11).

These results demonstrate that post-irradiation influx of BM-derived myelomonocytic cells into tumors may be inhibited by HIF-1α antagonists.

### 1. Summary of experimental results

The results of the above experiments demonstrated that CD11b+ myelomonocytic cells were present in the infiltrates of tumors that grow on irradiated tissues and irradiated tumors, and that these CD11b+ myelomonocytic cells expressed MMP-9 and responded to HIF-1α upregulation. Genetic and pharmaceutical/chemical depletion of MMP-9 abrogated tumor vasculogenesis, demonstrating that MMP-9 is associated with tumor grow in irradiated tissues.

CD11b+ myelomonocytic cells, including CD11b⁺Gr-1⁺ myeloid suppressor cells, have been recognized for their roles in promoting tumor progression, with various studies showing that these cells enhance tumor angiogenesis (Yang *et al.,* 2004), prepare sites of metastasis in the lung (Hiratsuka et al. (2006) Nat. Cell. Biol. 8:1369-75), and are responsible for the refractory nature of certain tumors to anti-VEGF treatment (Shojaei et al. (2007) Nat. Biotechnol. 8:911-20). BM-derived myelomonocytic cells may promote tumor growth in a positive feedback loop that involves other components of the tumor microenvironment (Allavena et al. (2005) Cancer Res. 65:2964-71; Lin et al. (2001) Mol. Cell. 26:63-74; Luo et al. (2006) J. Clin. Invest. 116:2132-41; Zeisberger et al. (2006) Br. J. Cancer 95:272-81), although variations in the recruitment of BM-derived cells in different tumors have resulted in inconclusive results.

Indeed, the present results suggest that BM-derived cells expressing MMP-9 are sufficient but not essential for tumor vasculogenesis in solid tumors, as evidenced by the similar rate of tumor growth on pre-irradiated tissues of WT + *MMP-9* KO BM compared to WT mice + WT BM. Non-BM cells such as fibroblasts and smooth muscle cells appear to express sufficient MMP-9 to compensate for a deficiency of MMP-9 from BM cells, explaining why clinical trials involving MMP inhibitors, either alone or in combination with cytotoxic agents, have been disappointing in terms of reducing the rate of tumor growth (Unsal et al. (2007) Int. J. Rad. Oncol. Biol. Phys. 67:196-203; Coussens et al. (2002) Cell 103:481-90).

However, none of these previous studies have been performed in conjunction with radiotherapy, which selectively inhibits local angiogenesis and make tumor growth dependent on vasculogenesis. The present experiments demonstrate that MMPs, particularly MMP-9, is required for tumor growth on irradiated tissues and tumor regrowth following irradiation of a tumor and the underlying tissues, and that inhibiting or removing the source of the MMP inhibits tumor growth.

While a number of exemplary aspects and embodiments have been discussed above, those skilled in the art will recognize certain modifications, permutations, additions and subcombinations thereof, are within the spirit and scope of the methods. All the references cited herein are indicative of the level of skill in the art and are hereby incorporated by reference in their entirety.

The present invention also retates to the following items:
1. A method for inhibiting post-irradiation tumor growth in a subject, comprising:
   administering to the subject an amount of an inhibitor of vasculogenesis selected from the group consisting of a matrix metalloproteinase (MMP) inhibitory compound, an agent that inactivates bone marrow (BM)-derived cells that express matrix metalloproteinase, and a HIF-1α inhibitor,
   wherein said administering occurs before, during, or after a subject is exposed to radiation therapy, and wherein said amount is a therapeutically effective to prevent or inhibit regrowth of a tumor after the subject is exposed to radiation therapy.
2. The method of item 1, wherein the inhibitor of vasculogenesis is an MMP inhibitory compound.
3. The method of item 2, wherein the MMP inhibitory compound is selected from the group consisting of an antibody or fragment thereof and a small molecule.
4. The method of item 3, wherein the MMP inhibitory compound is selective for MMP-9.
5. The method of item 1, wherein the inhibitor of vasculogenesis is an agent that inactivates BM-derived cells that express matrix metalloproteinase.
6. The method of item 5, wherein the BM-derived cells that express a matrix metalloproteinase in the tumors are CD11b-expressing myelomonocytic cells.
7. The method of item 6, wherein the agent is selected from the group consisting of a neutralizing antibody specific for a dimer comprising CD11b, an agent that inhibits CXCR4, AMD3100, and carrageenan.
8. The method of item 7, wherein the agent is a neutralizing antibody specific for CD116.
9. The method of item 8, wherein the agent is a neutralizing antibody specific for a dimer comprising CD11b and is specific for CD18.
10. The method of item 7, wherein the agent inhibits CXCR4.
11. The method of item 10, wherein the agent inhibits CXCR4 and SDF1 binding.
12. The method of item 7, wherein the agent is AMD3100.
13. The method of item 7, wherein the agent is carrageenan.
14. The method of item 1, wherein the inhibitor of vasculogeneis is a HIF-1α inhibitor.
15. The method of item 14, wherein the HIF-1α inhibitor is NSC 134754.
16. The method of item 7, wherein the MMP is MMP-9.
17. The method of item 1, wherein the tumor is selected from the group consisting of a head or neck tumor, a tumor of the mouth, and glioblastoma.
18. The method of item 1, wherein the inhibitor of vasculogenesis is administered systemically.
19. The method of item 1, wherein the inhibitor of vasculogenesis is surgically implanted underneath the skin.
20. The method of item 1, wherein the inhibitor of vasculogenesis is administered intratumorally.

## Claims

1. A composition for inhibiting re-growth of a solid tumor following radiation treatment, wherein said agent is to be administered before, during or after radiation exposing the tumor to radiation therapy, the composition comprising an agent that inactivates MMP expressing bone marrow (BM)-derived cells.

2. A composition for preventing MMP expressing bone marrow (BM)-derived cells infiltration into solid tumor following radiation treatment, wherein said agent is to be administered before, during or after radiation exposing the tumor to radiation therapy, the composition comprising an agent that inactivates MMP expressing bone marrow (BM)-derived cells.

3. The composition according to claims 1 or 2, wherein the MMP expressing bone marrow (BM)-derived cells are CD11b-expressing myelomonocytic cells.

4. The composition according to claims 1, 2 or 3, wherein the agent is an agent that is specific to an antigen on the matrix metalloproteinase (MMP) expressing bone marrow (BM)-derived cells.

5. The composition according to any of the preceding claims, wherein the agent inhibits CXCR4 and SDF1 binding.

6. The composition according to claim 5, wherein the agent inhibits CXCR4.

7. The composition according to claim 6, wherein the agent is an antibody.

8. The composition according to claim 7, wherein the antibody is directed to CXCR4.

9. The composition according to claims 4 or 5, wherein the agent is a small molecule antagonist of CXCR4.

10. The composition according to claim 4, wherein the agent is specific for CD11b or CD18.

11. The composition according to claims 2 or 3, wherein the agent is a HIF-1α inhibitor.

12. The composition of any of the preceding claims, wherein the tumor is selected from the group consisting of a head or neck tumor, a tumor of the mouth, and glioblastoma.

13. The composition of any of the preceding claims, wherein the composition is administered before, during or after a subject is exposed to irradiation therapy.

14. The composition of any of the preceding claims, wherein the composition is administered in a therapeutically effective amount to prevent or inhibit regrowth of a tumor after the subject is exposed to radiation therapy.

15. The composition of any of the preceding claims, wherein the composition is administered systemically, is surgically implanted underneath the skin or is administered intratumorally.
